(19) Europäisches Patentamt
European Patent Office
Office européen des brevets

(11) EP 3 101 142 B1

(12) **EUROPEAN PATENT SPECIFICATION**

(45) Date of publication and mention of the grant of the patent:
**05.12.2018 Bulletin 2018/49**

(51) Int Cl.:
***C12Q 1/6883*** *(2018.01)*

(21) Application number: **16172768.0**

(22) Date of filing: **03.06.2016**

(54) **METHOD AND DEVICE FOR ASSISTING DIAGNOSIS OF EFFICACY OF METHOTREXATE IN PATIENT WITH RHEUMATOID ARTHRITIS**

VERFAHREN UND VORRICHTUNG ZUR UNTERSTÜTZUNG DER WIRKSAMKEIT VON METHOTREXAT BEI PATIENTEN MIT RHEUMATOIDER ARTHRITIS

PROCÉDÉ ET DISPOSITIF POUR FACILITER LE DIAGNOSTIC DE L'EFFICACITÉ DU MÉTHOTREXATE CHEZ LES PATIENTS ATTEINTS DE POLYARTHRITE RHUMATOÏDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.06.2015 JP 2015114056**

(43) Date of publication of application:
**07.12.2016 Bulletin 2016/49**

(73) Proprietors:
- **SYSMEX CORPORATION**
  **Kobe-shi**
  **Hyogo 651-0073 (JP)**
- **Shinko Medical Corporation**
  **Kobe-shi, Hyogo 651-0072 (JP)**

(72) Inventor: **Kumagai, Shunichi**
**Amagasaki-shi, Hyogo 661-0953 (JP)**

(74) Representative: **Paemen, Liesbet R.J. et al**
**De Clercq & Partners**
**Edgard Gevaertdreef 10 a**
**9830 Sint-Martens-Latem (BE)**

(56) References cited:
**WO-A2-2005/022118**

- **WESSELS JUDITH A M ET AL: "A clinical pharmacogenetic model to predict the efficacy of methotrexate monotherapy in recent-onset rheumatoid arthritis", ARTHRITIS & RHEUMATISM, vol. 56, no. 6, 1 June 2007 (2007-06-01), pages 1765-1775, XP002458564, WILEY INTERSCIENCE, US ISSN: 0004-3591, DOI: 10.1002/ART.22640**
- **H. HAYASHI ET AL: "Genetic polymorphisms in folate pathway enzymes as a possible marker for predicting the outcome of methotrexate therapy in Japanese patients with rheumatoid arthritis", JOURNAL OF CLINICAL PHARMACY AND THERAPEUTICS., vol. 34, no. 3, 1 June 2009 (2009-06-01), pages 355-361, XP055310597, GB ISSN: 0269-4727, DOI: 10.1111/j.1365-2710.2009.01046.x**
- **DATABASE dbSNP [Online] XP002762895, retrieved from NCBI Database accession no. rs1544105**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 101 142 B1

**Description**

TECHNICAL FIELD

The present invention relates to a method and a device for assisting diagnosis of efficacy of methotrexate in a patient with rheumatoid arthritis.

BACKGROUND

In rheumatoid arthritis therapy, methotrexate (MTX) known as a folic acid antagonist is currently regarded as a first-line drug. MTX plays a major role in controlling the symptoms of rheumatoid arthritis since a potent immunosuppressive action of MTX prevents joint destruction. Generally, the therapeutic effect of MTX increases depending on the amount of MTX used. However, in practice, the efficacy of MTX varies considerably from individual to individual. It is desired to treat each patient with rheumatoid arthritis with MTX in an optimal amount so as to ensuree a high therapeutic effect. However, it is very difficult to predict the efficacy of MTX in the patients.

In recent years, attention has been paid to the fact that the blood concentration of MTX polyglutamate (MTX-PG), which is a metabolic product of MTX, is associated with the efficacy of MTX. For example, Davila L. and Ranganathan P., Nat. Rev. Rheumatol, vol.7, p.537-550 (2011) and Wessels J. A. M. et al., Arthritis Rheum, vol.56, p.1765-1775 (2007) report that protein molecules of SLC19A1 (also referred to as "RFC-1"), GGH, and FPGS are involved in the metabolism between MTX and MTX-PG. Specifically, SLC19A1 is a transporter for MTX into cells, GGH is an enzyme that polyglutamylates MTX and converts it into MTX-PG, and FPGS is an enzyme that depolyglutamylates MTX-PG and converts it into MTX. Hayashi H. et al., J. Clin. Pharmacy Therapeutics, vol. 34, p.355-361 (2009) reports that the genetic polymorphisms of GGH and SLC19A1 genes can be used to determine the efficacy of MTX.

SUMMARY OF THE INVENTION

In the case where the efficacy of MTX for each patient can be determined, the determination results thereof provide a useful indication for deciding the therapeutic strategy for rheumatoid arthritis. In view of the reports, the present inventors have analyzed SNPs of the SLC19A1, GGH, and FPGS genes associated with MTX metabolism and attempted to determine the efficacy of MTX based on the analysis results thereof.

However, contrary to their expectations, they could not obtain precise determination results as to the efficacy. Accordingly, there is a need for further development of an SNP marker set that enables the efficacy of MTX to be determined more precisely.

Therefore, the present invention provides a method for assisting diagnosis of efficacy of MTX in a patient with rheumatoid arthritis, the method comprising the steps of: analyzing the single nucleotide polymorphism (SNPs) of the FPGS gene FPGS c.64A>G and either one of FPGS c.*192A>G and rs1544105, the SNP of the FMO2 gene FMO2 c.585A>G, the SNP of the SLC28A3 gene SLC28A3 c.267G>A, the SNP of the EPHX1 gene EPHX1 c.357G>A, and the SNP of the PPARG gene PPARG c.*1411G>C in a biological sample obtained from a patient with rheumatoid arthritis; obtaining a numerical index associated with the efficacy of MTX from the analysis result; comparing the obtained numerical index with a predetermined threshold; and determining the efficacy of MTX in the patient based on the comparison results.

In particular embodiments, the SNPs of the GGH gene GGH c.452C>T, and either one of GGH c.-401C>T, or GGH c.16T>C, is further analyzed. In a further embodiment, one of the SNPs of the SLC19A1 gene selected from SLC19A1 c.696T>C and SLC19A1 c.80G>A is further analyzed in the analysis step.

In particular embodiments, the numerical index associated with the efficacy of MTX is a predictive value calculated by a multiple logistic model. In particular embodiments, when the predictive value is more than or equal to the predetermined threshold in the determining step, the efficacy of MTX in the patient is determined to be high. In particular embodiments, when the predictive value is lower than the predetermined threshold, the efficacy of MTX in the patient is determined to be low.

Another aspect of the present invention is a device for determining efficacy of methotrexate in a patient with rheumatoid arthritis. The device comprises a computer. The computer comprises a processor and a memory under control of the processor. The memory stores a computer program that allows the computer to execute the steps of: analyzing in a biological sample obtained from a patient with rheumatoid arthritis: the single nucleotide polymorphism (SNPs) of the FPGS gene FPGS c.64A>G and either one of FPGS c.*192A>G and rs1544105, the SNP of the FMO2 gene FMO2 c.585A>G, the SNP of the SLC28A3 gene SLC28A3 c.267G>A, the SNP of the EPHX1 gene EPHX1 c.357G>A, and the SNP of the PPARG gene PPARG c.*1411G>C; and obtaining a numerical index associated with the efficacy of MTX from the analysis result; comparing the obtained numerical index with a predetermined threshold; and determining the efficacy of MTX in the patient based on the comparison results. In particular embodiments, the SNPs of the GGH gene GGH c.452C>T, and either one of GGH c.-401C>T, or GGH c.16T>C, are analyzed. In a further embodiment, one of the SNPs of the SLC19A1 gene selected from SLC19A1 c.696T>C and SLC19A1 c.80G>A is further analyzed in the analysis step.

Diagnosis of MTX efficacy for a rheumatoid arthritis patient can be assisted by the present invention.

BRIEF DESCRIPTION OF THE DRAWINGS

Fig. 1 is a schematic view illustrating an example of a determination device for determining the efficacy of MTX.

Fig. 2 is a block diagram illustrating a hardware configuration of the determination device illustrated in Fig. 1.

Fig. 3 is a flow chart for determining the efficacy of MTX using the determination device illustrated in Fig. 1.

Fig. 4 is an ROC curve when the efficacy of MTX has been determined using Discriminant 1.

Fig. 5 is an ROC curve when the efficacy of MTX has been determined using Discriminant 2.

Fig. 6 is an ROC curve when the efficacy of MTX has been determined using Discriminant 3.

Fig. 7 is an ROC curve when the efficacy of MTX has been determined using Discriminant 4.

Fig. 8 is an ROC curve when the efficacy of MTX has been determined using Discriminant 5.

Fig. 9 is an ROC curve when the efficacy of MTX has been determined using Discriminant 6.

Fig. 10 is an ROC curve when the efficacy of MTX has been determined using Discriminant 7.

Fig. 11 is a schematic view illustrating an example of a reagent kit.

DETAILED DESCRIPTION OF THE PREFERRED EMBODIMENTS

In a method for determining efficacy of methotrexate in patients with rheumatoid arthritis as envisaged herein, at least one of SNPs of at least one gene selected from FPGS gene, GGH gene, and SLC19A1 gene, at least one of SNPs of FMO2 gene, at least one of SNPs of SLC28A3 gene, at least one of SNPs of EPHX1 gene, and at least one of SNPs of PPARG gene in a biological sample obtained from a patient with rheumatoid arthritis are first analyzed.

SNP is a kind of genetic polymorphism, and means a state that only one site of the base sequence of genomic DNA is mutated. The SNP is different from a point mutation in that the mutation is observed in a predetermined population at a frequency of 1% or more. The SNP used herein may also be referred to as "mutation at the genetic polymorphism site".

The patient with rheumatoid arthritis (hereinafter, also referred to as "patient with RA") is not particularly limited as long as the patient is diagnosed as having rheumatoid arthritis. The method is related to the determination of the efficacy of MTX. Thus, a patient with RA who has not been treated by MTX therapy yet and a patient with RA to whom MTX has already been administered, but the efficacy of MTX has not been found are preferred as subjects of the method envisaged herein. Consequently, the method envisaged herein can also be interpreted as "a method for predicting efficacy of MTX in a patient with RA".

In the methods envisaged herein, the biological sample is not particularly limited as long as it is a sample from living body which contains genomic DNA of a patient with RA. Examples of the biological sample include body fluid, urine, and tissues and cells obtained by operations or biopsies. Examples of the body fluid include blood, plasma, serum, synovial fluid, lymph fluid, ascitic fluid, bone marrow fluid, and nipple discharge. From the viewpoint of easy collection, peripheral blood is preferred as the biological sample.

In the methods envisaged herein, it is preferable to prepare a measurement sample by extracting the genomic DNA from the biological sample. Methods for extracting the genomic DNA from the biological sample are well known in the art. For example, the genomic DNA can be extracted by mixing the biological sample with a treatment liquid containing a surfactant (e.g., sodium cholate or sodium dodecyl sulfate), subjecting the obtained mixture liquid to a physical treatment (stirring, homogenization or ultrasonic fragmentation), and allowing the genomic DNA contained in the biological sample to be free in the mixture liquid. In this case, a supernatant containing the genomic DNA released by centrifuging the mixture liquid to precipitate cell debris is preferably used in a later-described detecting step. The obtained supernatant may be purified by any well-known method in the art. The genomic DNA can also be extracted from the biological sample and purified by using a commercially-available kit.

In the methods envisaged herein, as long as there is a region where the genetic polymorphism site to be detected is present, cDNA or cRNA synthesized from the genomic DNA of the patient with RA may be used as a measurement sample. The method itself to synthesize cDNA or cRNA from the genomic DNA is well known in the art. Hereinafter, the genomic DNA obtained from the biological sample of the patient with RA as well as cDNA and cRNA obtained from this genomic DNA are collectively referred to as "nucleic acid derived from the biological sample of the patient with RA".

The names of each of the genes are shown in Table 1 below. The SLC19A1 gene is also referred to as "reduced folate carrier-1 (RFC-1)". Regarding these genes, the base sequences themselves of genomic DNA including SNPs to be analyzed are known. These base sequences can be obtained from a well-known database provided by, for example, the National Center for Biotechnology Information (NCBI) (http://www.ncbi.nlm.nih.gov/).

[Table 1]

| Gene symbol | Gene name |
|---|---|
| FPGS | folypolyglutamate synthase |
| GGH | gamma glutamyl hydrolase |
| SLC19A1 | solute carrier family 19, member 1 |
| FMO2 | flavin containing monooxygenase 2 |
| SLC28A3 | solute carrier family 28 (sodium-coupled nucleoside transporter), member 3 |
| EPHX1 | epoxide hydrolase 1, microsomal (xenobiotic) |
| PPARG | peroxisome proliferator-activated receptor gamma |

In the methods envisaged herein, the combination of genes having SNPs to be analyzed is not particularly limited as long as it is a combination of at least five genes including at least one gene selected from FPGS gene, GGH gene, and SLC19A1 gene and four genes (FMO2 gene, SLC28A3 gene, EPHX1 gene, and PPARG gene). The at least one gene selected from FPGS gene, GGH gene, and SLC19A1 gene is not particularly limited and it is preferable to include the FPGS gene.

In the method envisaged herein, the position and number of SNPs to be analyzed on each of the genes are not particularly limited, and can be appropriately selected from known SNPs as to each of the genes. Regarding each of the genes, it is preferable to analyze SNPs at the sites shown below. The site of the FPGS gene is at least one position selected from c. 64 position, c.*192 position, and g. 130562725 position and preferably c. 64 position. The site of the GGH gene is at least one position selected from c. 452 position, c.-401 position, and c.16 position. The site of the SLC19A1 gene is c. 696 position and c.80 position. The site of the FMO2 gene is c. 585 position. The site of the SLC28A3 gene is c. 267 position. The site of the EPHX1 gene is c. 357 position. The site of the PPARG gene is c. *1411 position.

The notation of the sites where SNPs are present in the genes will be hereinafter described. This notation is based on the guideline defined by the Human Genome Variation Society (http://www.hgvs.org/).

The c.64 position of the FPGS gene indicates the position of the 64th base counting from the 5' end of the coding sequence of the FPGS gene to the downstream side. The term "counting from the 5' end of the coding sequence of gene to the downstream side" means that bases in the base sequence of gene on genomic DNA are counted from the 1st base: adenine of the start codon (ATG) located at the 5' end of the coding sequence of gene, to the downstream side (3' side).

The FPGS c.*192 position indicates the position of the 192nd base counting from the 3' end of the coding sequence of the FPGS gene to the downstream side. The term "counting from the 3' end of the coding sequence of gene to the downstream side" means that bases in the base sequence of gene on genomic DNA are counted from the 1st base: the base adjacent to the 3' side of the base at the 3' end of the coding sequence of gene, to the downstream side (3' side).

The GGH c.452 position indicates the position of the 452nd base counting from the 5' end of the coding sequence of the GGH gene to the downstream side.

The GGH c.-401 position indicates the position of the 401st base counting from the 5' end of the coding sequence of the GGH gene to the upstream side. The term "counting from the 5' end of the coding sequence of gene to the upstream side" means that bases in the base sequence of gene on genomic DNA are counted from the 1st base: the base adjacent to the 5'side of the adenine of the start codon (ATG) located at the 5' end of the coding sequence of gene, to the upstream side (5' side).

The GGH c.16 position indicates the position of the 16th base counting from the 5' end of the coding sequence of the GGH gene to the downstream side.

The SLC19A1 c.696 position indicates the position of the 696th base counting from the 5' end of the coding sequence of the SLC19A1 gene to the downstream side.

The SLC19A1 c.80 position indicates the position of the 80th base counting from the 5' end of the coding sequence of the SLC19A1 gene to the downstream side.

The c.585 position of the FMO2 gene indicates the position of the 585th base counting from the 5' end of the coding sequence of the FMO2 gene to the downstream side.

The c. 267 position of the SLC28A3 gene indicates the position of the 267th base counting from the 5' end of the coding sequence of the SLC28A3 gene to the downstream side.

The c.357 position of the EPHX1 gene indicates the position of the 357th base counting from the 5' end of the coding sequence of the EPHX1 gene to the downstream side.

The c.*1411 position of the PPARG gene indicates the position of the 1411st base counting from the 3' end of the coding sequence of the PPARG gene to the downstream side.

In the method envisaged herein, the SNP of the FPGS gene is preferably at least one selected from FPGS c.64A>G, FPGS c.*192A>G, and FPGSg.130562725G>A. Particularly, it is preferable to analyze FPGS c.64A>G. Multicollinearity is observed between FPGS g.130562725G>A and FPGS c.*192A>G. Thus, it is more preferable to analyze only one of them. The selection of either one of FPGS g.130562725G>A and FPGS c.*192A>G is not particularly limited.

The SNP of the GGH gene is preferably at least one selected from GGH c.452C>T, GGH c.-401C>T, and GGH c.16T>C. Multicollinearity is observed between GGH c.16T>C and GGH c.-401C>T. Thus, it is more preferable to analyze only one of them. The selection of either one of GGH c.16T>C and GGH c.-401C>T is not particularly limited.

The SNP of the SLC19A1 gene is preferably at least one selected from SLC19A1 c.696T>C and SLC19A1 c.80G>A. Multicollinearity is observed between SLC19A1 c.696T>C and SLC19A1 c.80G>A. Thus, it is more preferable to analyze only one of them. The selection of either one of SLC19A1 c.696T>C and SLC19A1 c.80G>A is not particularly limited.

The SNP of the FMO2 gene is preferably FMO2 c.585A>G. The SNP of the SLC28A3 gene is preferably SLC28A3 c.267G>A. The SNP of the EPHX1 gene is preferably EPHX1 c.357G>A. The SNP of the PPARG gene is preferably PPARG c.*1411G>C.

In the notation of the SNPs, the base before mutation and the base after mutation are shown at the left and right sides of the symbol ">", respectively. For example, "FPGS c.64A>G" represents a mutation from adenine to guanine at the c.64 position of the FPGS gene.

The SNP positions on genomic DNA can be obtained from a well-known database provided by NCBI (http://www.ncbi.nlm.nih.gov/). The RS numbers (Reference SNP ID number) of the SNPs are shown in Table 2. SEQ ID NOs: 1 to 12 in Table 2 represent sequences of 1001 bases in total which include 500 bases in front of the SNPs and 500 bases behind the SNPs. Hence, in the base sequences represented by SEQ ID NOs: 1 to 12, the position of the 501st base is a position of SNP. In the base sequences represented by SEQ ID NOs: 1 to 12, the base (501st base) in the position of SNP represents the base before mutation.

[Table 2]

| SNP | RS NUMBER | SEQ ID NO: |
|---|---|---|
| FPGS c.64A>G | rs10760502 | SEQ ID NO: 1 |
| FPGS c.*192A>G | rs10106 | SEQ ID NO: 2 |
| FPGS g.130562725G>A | rs1544105 | SEQ ID NO: 3 |
| GGH c.452C>T | rs11545078 | SEQ ID NO: 4 |
| GGH c.-401C>T | rs3758149 | SEQ ID NO: 5 |
| GGH c.16T>C | rs1800909 | SEQ ID NO: 6 |
| SLC19A1 c.696T>C | rs12659 | SEQ ID NO: 7 |
| SLC19A1 c.80G>A | rs1051266 | SEQ ID NO: 8 |
| FMO2 c.585A>G | rs2020861 | SEQ ID NO: 9 |
| SLC28A3 c.267G>A | rs7867504 | SEQ ID NO: 10 |
| EPHX1 c.357G>A | rs1131873 | SEQ ID NO: 11 |
| PPARG c.*1411G>C | rs1152003 | SEQ ID NO: 12 |

The SNP analysis may be to determine the presence or absence of SNP, i.e., whether or not any mutation is present at the genetic polymorphism site. The SNP analysis may be to obtain the number of SNPs in the allelic gene, or may be to obtain a value correlated with the presence or absence of SNP. The value correlated with the presence or absence of SNP may be, for example, a measured value in itself to be obtained by the SNP analysis unit described later, or may be a value obtained based on the measured value. The value obtained based on the measured value is not particularly limited, and examples thereof include sums, differences, products, ratios, logarithms, and statistically representative values (e.g., mean or median). The value obtained based on the measured value may be a value that is calculated from the measured value based on a predetermined formula or the like.

The SNP analysis unit is not particularly limited in the method envisaged herein, and it may be appropriately selected from SNP detection and SNP typing methods well known in the art. Examples of the method include direct sequencing methods, quenching probe (QProbe) methods (refer to Japanese Patent No. 4950414), and microarray methods. A commercially available SNP typing kit such as TaqMan (trademark) SNP Genotyping Assays may be used.

In the case of a direct sequencing method, the presence or absence of SNP is detected by amplifying a region including SNPs in genomic DNA by PCR assay using a predetermined primer set, and analyzing the base sequence of the obtained amplified products. In all the genes shown in Table 1, the base sequences themselves of genomic DNA including SNPs to be analyzed are well known. Accordingly, primers for amplification and primers for sequencing may be designed based on the base sequences. The primers can be synthesized by any nucleic acid synthesis method well known in the art.

In the case of detecting SNP by a QProbe method, a region including SNPs in genomic DNA is amplified by PCR assay using a predetermined primer set, followed by hybridization with a QProbe having a sequence complementary to the base sequence of the obtained amplified product. When SNPs are present in the amplified products, the temperature causing dissociation of the amplified products and the QProbe is different from the temperature when no SNP is present in the amplified products. Thus, this difference is utilized to detect SNPs in the QProbe method. The QProbe in itself is well known in the art and is commonly available.

In the case of detecting SNP by a microarray method, the microarray for analysis may be prepared by immobilizing on a substrate a nucleic probe complementary to the base sequence of a region of SNP present. The microarray can be prepared according to a well-known method in the art. In the analysis using a microarray, a nucleic acid derived from a biological sample is preferably labeled with a labeling substance well known in the art. Examples of the labeling substance include fluorescent substances, haptens such as biotin, and radioactive substances. Examples of the fluorescent substances include Cy3, Cy5, FITC, and Alexa Fluor (trademark). Labeling of DNA facilitates measurement of a signal from a probe on the microarray. The method for labeling DNA with the labeling substance is well known in the art.

The above signal may be any suitable signal depending on the type of the microarray. For example, the signal may be an electric signal generated when a DNA fragment hybridizes to each probe on the microarray or may be a fluorescence or luminescence signal generated from a labeling substance when the nucleic acid derived from a biological sample is labeled. The signal can be detected using a scanner included in a normal microarray measurement apparatus The scanner is, for example, GeneChip (registered trademark) Scanner3000 7G (Affymetrix, Inc.).

In particular embodiments of the methods envisaged herein, the efficacy of MTX in a patient with RA is determined based on the analysis results obtained in the analysis step.

For example, it may be determined whether or not the efficacy of MTX in the patient with RA is high based on the analysis results. The determination of the efficacy of MTX can be interpreted as prediction of the therapeutic effect of MTX on the patient with RA or prediction of the therapeutic reactivity with MTX in the patient with RA. For example, when the efficacy of MTX is determined to be high, it is possible to predict that the therapeutic effect of MTX on the patient is high.

On the other hand, when the efficacy of MTX is determined to be low, it is possible to predict that the therapeutic effect of MTX on the patient is low.

It is preferable that a numerical index associated with the efficacy of MTX is obtained from the analysis results, and the obtained numerical index is compared with a predetermined threshold, and the efficacy of MTX in the patient with RA is determined based on the comparison results. Examples of the numerical index associated with the efficacy of MTX include numerical indices obtained by multivariate analysis of values correlated with the presence or absence of SNP as to each of the genes, the number of SNP or the presence or absence of SNP. Examples of the numerical indices obtained by multivariate analysis include predictive values calculated by a multiple logistic model. The multivariate analysis in itself is well known in the art and is executed by a computer through the use of commercially available software.

The determination can be performed using a regression equation constructed based on the multiple logistic model. As the regression equation, for example, the regression equation of Formula (I) below can be used. The regression equation of Formula (I) is based on the continuous scale. The determination is performed by comparing a P value obtained by assigning the information on each of the analyzed SNPs to Formula (I) with a predetermined threshold.

As the SNP information, "0" can be used when there are no mutations at all in the genetic polymorphism sites of allelic genes in the locus, "1" can be used when there is a mutation at a genetic polymorphism site of one of the allelic genes, and "2" can be used when there are mutations at genetic polymorphism sites of both the allelic genes. These values are assigned to $*SNP_n$ of Formula (I) below. $b_0$ represents a coefficient of a piece, $b_1$ to $b_n$ represent coefficients of the SNPs, and these coefficients can be appropriately set depending on the type of SNP used for discrimination.
[Equation 1]

$$P = \frac{1}{1+\exp[-(b_0+b_1*SNP_1+b_2*SNP_2+\cdots+b_n*SNP_n)]} \quad (I)$$

Alternatively, the regression equation of Formula (II) below may be used as the regression equation. The regression equation of Formula (II) is based on the nominal scale. The determination is performed by comparing a P value obtained by Formula (II) with a predetermined threshold using the information on each of the analyzed SNPs. In Formula (II), the SNP information is designated as "WT" when there are no mutations at all in the genetic polymorphism sites of allelic genes in the locus, the SNP information is designated as "HT when there is a mutation at a genetic polymorphism site of one of the allelic genes, and the SNP information is designated as "MT" when there are mutations at genetic polymorphism sites of both the allelic genes. In Formula (II), values to be assigned to $b_1$ to $b_n$ (which are coefficients of the SNPs) vary depending on the SNP information, i.e., the type of SNP used for discrimination and the genotype thereof (WT, HT or MT). The values to be assigned to $b_1$ to $b_n$ can be calculated by any known method in the art. In Formula (II), $b_0$ represents a coefficient of a piece.
[Equation 2]

$$P = \frac{1}{1+\exp[-(b_0+b_1+b_2+\cdot\cdot\cdot+b_n)]} \quad (II)$$

The P value calculated by Formula (I) or (II) above represents a predictive value of the efficacy of MTX (hereinafter, also referred to as "predictive value of efficacy"). In the embodiment, when a predetermined threshold is set to, for example, 0.5 and the predictive value of efficacy is 0.5 or more, the efficacy of MTX in a patient with RA can be determined to be high (or the therapeutic effect of MTX is high). When the predictive value of efficacy is lower than 0.5, the efficacy of MTX in a patient with RA can be determined to be low (or the therapeutic effect of MTX is low). The predetermined threshold can be optionally set by those skilled in the art. The regression equations (I) and (II) may be used as discriminants of the predictive value of efficacy in the method according to the embodiment.

The present disclosure encompasses a method for assisting diagnosis of efficacy of MTX in a patient with RA. This method provides the determination results of the efficacy of MTX to a physician or the like so that diagnosis of the efficacy of MTX by a physician or the like can be assisted. That is, the physician or the like diagnoses the efficacy of MTX in the patient with RA based on the determination results of the efficacy of MTX and then can determine whether or not MTX is administered to the patient. In the case of a patient with RA to whom MTX has already been administered, the physician or the like can determine whether or not the administration of MTX is continued or the dosage of MTX is changed.

The present disclosure also encompasses a device for determining the efficacy of MTX in a patient with RA. For example, the device is as follows.

A device for determining the efficacy of MTX in a patient with rheumatoid arthritis, the device comprising a computer. The computer comprises a processor and a memory under control of the processor. The memory stores a computer program that allows the computer to execute the steps of:

analyzing at least one of single nucleotide polymorphisms (SNPs) of at least one gene selected from FPGS gene, GGH gene, and SLC19A1 gene, at least one of SNPs of FMO2 gene, at least one of SNPs of SLC28A3 gene, at least one of SNPs of EPHX1 gene, and at least one of SNPs of PPARG gene in a biological sample obtained from a patient with rheumatoid arthritis; and
determining whether the efficacy of methotrexate (MTX) in the patient with rheumatoid arthritis is high based on the obtained analysis results.

The present disclosure also encompasses a computer program to allow a computer to execute determination of the efficacy of MTX in a patient with RA. For example, the computer program is as follows.

A computer program to allow a computer to execute determination of the efficacy of MTX in a patient with RA, wherein the program stored in a computer readable medium allows the computer to execute the steps of:

analyzing at least one of single nucleotide polymorphisms (SNPs) of at least one gene selected from FPGS gene, GGH gene, and SLC19A1 gene, at least one of SNPs of FMO2 gene, at least one of SNPs of SLC28A3 gene, at least one of SNPs of EPHX1 gene, and at least one of SNPs of PPARG gene in a biological sample obtained from a patient with rheumatoid arthritis; and

determining whether or not the efficacy of methotrexate (MTX) in the patient with rheumatoid arthritis is high based on the obtained analysis results.

The medium may be a medium in which the computer program is non-temporarily stored and is readable by the computer.

Hereinafter, an example of a suitable device for carrying out the method of the embodiment will be described with reference to the drawings. However, the embodiment is not limited only to this example. Fig. 1 is a schematic view illustrating an example of a determination device for determining the efficacy of MTX. A determination device 10 illustrated in Fig. 1 includes a measurement device 20 and a computer system 30 connected to the measurement device 20.

The measurement device 20 is a microarray scanner that detects a signal based on a nucleic acid bound to a probe on a microarray. The signal is optical information such as a fluorescent signal. When the microarray being in contact with a measurement sample is mounted onto the measurement device 20, the measurement device 20 obtains optical information based on a nucleic acid of a biological sample derived from a patient with RA which is bound to a probe on a microarray, and sends the obtained optical information to the computer system 30.

The microarray scanner is not particularly limited as long as it can detect the signal based on a nucleic acid bound to a probe on a microarray. The signal changes corresponding to the labeling substance used for labeling the nucleic acid of the biological sample derived from the patient with RA. Thus, the microarray scanner can be appropriately selected depending on the type of the labeling substance. For example, when the labeling substance is a radioactive substance, a microarray scanner that can detect radioactive rays emitted from the radioactive substance is used as the measurement device 20.

In the case of detecting SNP by the direct sequencing method, the measurement device 20 may be a device including a nucleic acid amplification device and a sequence analysis device. In this case, a measurement sample and a reaction liquid containing enzymes and primers for nucleic acid amplification are mounted in the measurement device 20, and the nucleic acid in the reaction liquid is amplified by the nucleic acid amplification method. Then, the measurement device 20 analyzes the base sequences of the amplification products, obtains sequence information, and sends the obtained sequence information to the computer system 30.

The computer system 30 includes a computer main body 300, an input unit 301, and a display unit 302 that displays information on specimen and determination results. The computer system 30 receives optical information from the measurement device 20. The processor of the computer system 30 executes program to determine the efficacy of MTX in a patient with RA based on the optical information. The computer system 30 may be a device different from the measurement device 20 as shown in Fig. 1, or may be a device that includes the measurement device 20. In the latter case, the computer system 30 may be the determination device 10 in itself.

As shown in Fig. 2, the computer main body 300 includes a central processing unit (CPU) 310, a read only memory (ROM) 311, a random access memory (RAM) 312, a hard disk 313, an input/output interface 314, a readout device 315, a communication interface 316, and an image output interface 317. The CPU 310, the ROM 311, the RAM 312, the hard disk 313, the input/output interface 314, the readout device 315, the communication interface 316, and the image output interface 317 are data-communicably connected by a bus 318. The measurement device 20 is communicably connected to the computer system 30 by the communication interface 316.

The CPU 310 can execute the program stored in the ROM 311 and the program loaded on the RAM 312. The CPU 310 calculates a predictive value of efficacy, reads out discriminant stored in the ROM 311, and determines the efficacy. The CPU 310 outputs the determination results and displays the results on the display unit 302.

The ROM 311 is configured to include mask ROM, PROM, EPROM, EEPROM, and the like. As described above, the ROM 311 stores program to be executed by the CPU310 and data used for the program. The ROM 311 may store a predetermined threshold and a multiple logistic model discriminant.

The RAM 312 is configured to include SRAM and DRAM. The RAM 312 is used to read out the programs recorded on the ROM 311 and the hard disk 313. The RAM 312 is also used as a working area of the CPU 310 when these programs are executed.

The hard disk 313 is installed with computer programs to be executed by the CPU 310, such as operating system and application program (computer program for determining the efficacy of MTX) as well as data used in executing the computer programs. The hard disk 313 may store a predetermined threshold and a multiple logistic model discriminant.

The readout device 315 is configured to include a flexible disk drive, a CD-ROM drive, and a DVD-ROM drive. The readout device 315 can read out programs or data recorded on a portable recording medium 40.

The input/output interface 314 is configured to include a serial interface such as USB, IEEE 1394 or RS-232C, a parallel interface such as SCSI, IDE or IEEE1284, and an analog interface including a D/A converter or an A/D converter.

The input unit 301 such as a keyboard or mouse is connected to the input/output interface 314. An operator can input various instructions to the computer main body 300 via the input unit 301.

The communication interface 316 is, for example, an Ethernet (registered trademark) interface. The computer main body 300 can send printing data to a printer via the communication interface 316.

The image output interface 317 is connected to the display unit 302 including LCD, CRT or the like. Thus, the display unit 302 can output a video signal according to the image data provided from the CPU 310. The display unit 302 displays the image (screen) according to the input video signal.

Subsequently, the procedure of determining the degree of efficacy of MTX by the determination device 10 will be described. Here, the case of obtaining a predictive value of efficacy from fluorescent information based on a nucleic acid of a biological sample derived from a patient with RA which is bound to a probe on a microarray by a multiple logistic model, and performing determination using the obtained predictive value of efficacy will be described as an example. However, the configuration of the device is not limited only to this example.

Referring to Fig. 3, the CPU 310 of the determination device 10 obtains fluorescent information from the measurement device 20 in step S101. Subsequently, in step S102, the CPU 310 calculates the fluorescence intensity from the obtained fluorescent information and stores it in the RAM 312. In step S103, the CPU 310 determines the presence or absence of mutations at genetic polymorphism sites from the fluorescence intensity stored in the RAM 312, and calculates a predictive value of efficacy P based on the multiple logistic model in accordance with the discriminant stored in the ROM 311 or the hard disk 313.

Thereafter, in step S104, the CPU 310 determines the degree of efficacy of MTX in a patient with RA using the calculated predictive value of efficacy and the predetermined threshold stored in the ROM 311 or the hard disk 313. When the predictive value of efficacy P is lower than the predetermined threshold, the process proceeds to step S105 and the CPU 310 stores the determination results showing that the efficacy of MTX in the patient with RA is low in the RAM 312. On the other hand, when the predictive value of efficacy P is not lower than the predetermined threshold (i.e., when the predictive value of efficacy P is more than or equal to the threshold), the process proceeds to step S106 and the CPU 310 stores the determination results showing that the efficacy of MTX in the patient with RA is high in the RAM 312.

In step S107, the CPU 310 outputs the determination results, displays the results on the display unit 302, or prints the results on a printer. Consequently, it is possible to provide a physician or the like with information for assisting determination of whether or not the efficacy of MTX in a patient with RA is high.

Various reagents for detecting the SNP are provided as a reagent kit for users shown in Fig. 11. This reagent kit includes a reagent in accordance with the method for detecting SNP. For example, this reagent kit includes a reagent container having oligonucleotides for detecting SNP such as primers and probes, a reagent container having labeling substances such as fluorescent substances, and a reagent container having nucleic acid-amplifying enzymes such as DNA polymerases. According to the detection method, the kit may include microarrays and reagents such as TaqMan (trademark) Probe. This reagent kit may include the package insert of the reagents. For example, the configuration of the reagent kit and detection protocols are described in this package insert. In Fig. 11, a reference numeral 50 denotes a reagent kit, reference numerals 51 to 53 denote reagent containers, a reference numeral 54 denotes a package insert, and a reference numeral 55 denotes a container box.

Hereinafter, the present invention will be described in detail with reference to examples; however the present invention is not limited to the examples.

EXAMPLES

Comparative Example 1

SNPs of FPGS, GGH, and SLC19A1 genes associated with MTX metabolism were analyzed, and an attempt was made to determine the efficacy of MTX in patients with RA based on the analysis results.

(1) Subjects

First, medical information of patients who were diagnosed as rheumatoid arthritis and on MTX therapy was searched. The following two groups of the patients (100 patients in total) were selected.

1. Patients (44 patients) to whom MTX was administered in an amount of 8 mg/week or less and who have an ALT or AST value in the blood test of 40 IU/L or more; and
2. Patients (56 patients) to whom MTX was administered in an amount of 8 mg/week or more and who have an ALT or AST value in the blood test of 40 IU/L or less.

Among the 100 patients, 49 patients who had a DAS (disease activity score) of 28 based on CRP (C-reactive protein) value (hereinafter, also referred to as "DAS28 (CRP)") and who were subjected to liver function measurement over time after MTX administration were selected as subjects. Twenty-four weeks after MTX administration, the disease activity in these patients was evaluated by DAS28 (CRP) and the efficacy of MTX was determined in accordance with the EULAR (European League Against Rheumatism) treatment response criteria. As a result, among 49 patients, 22 patients belonged to the effective group (good), and 27 patients belonged to the ineffective group (moderate and none).

(2) Biological Samples

The peripheral blood of the subjects (49 subjects) was collected as a biological sample. The QIAamp DNA blood mini kit (QIAGEN) or QuickGene-810 (Kurabo Industries Ltd.) was used to extract genomic DNA in blood cells from the peripheral blood (200 μL). The extracted genomic DNA was stored at -30°C until it was used for genetic polymorphism analysis as described later.

(3) Gene Polymorphism Analysis

With respect to genomic DNA of each of the subjects, 8 SNPs present in the 3 genes were analyzed using TaqMan (trademark) SNP Genotyping Assays (Life Technologies Japan Ltd.). The 8 SNPs as well as Assay IDs of the kits used for analysis thereof are shown in Table 3.

[Table 3]

| SNP | SNP ANALYSIS METHOD |
|---|---|
| FPGS c.64A>G | TaqMan® SNP Genotyping Assays (Assay ID: AHPAVIP) |
| FPGS c.*192A>G | TaqMan® SNP Genotyping Assays (Assay ID: C_1459148_10) |
| FPGS g. 130562725G>A | TaqMan® SNP Genotyping Assays (Assay ID: C_8342611_10) |
| GGH c.452C>T | TaqMan® SNP Genotyping Assays (Assay ID: C_25623170_10) |
| GGH c.-401C>T | TaqMan® SNP Genotyping Assays (Assay ID: C_27512347_10) |
| GGH c.16T>C | TaqMan® SNP Genotyping Assays (Assay ID: C_8894713_10) |
| SLC19A1 c.696T>C | TaqMan® SNP Genotyping Assays (Assay ID: C_9520092_10) |
| SLC19A1 c.80G>A | TaqMan® SNP Genotyping Assays (Assay ID: AHQJTOX) |

A customized product of TaqMan (trademark) SNP Genotyping Assays which had been produced by Life Technologies Japan Ltd. on a commission basis was used to analyze FPGS c.64A>G and SLC19A1 c.80G>A. The primers and base sequences of TaqMan Probes included in this product are shown below.

Primers for analyzing FPGS c.64A>G and probes

- Forward primer: 5'- CGCGCCGCTCTATTCCT -3' (SEQ ID NO: 13)
- Reverse primer: 5'- GGCCACGCGCTCAAG -3' (SEQ ID NO: 14)
- TaqMan Probe Reporter 1: 5'- CGCGGCATAACGA -3' (SEQ ID NO: 15)
- TaqMan Probe Reporter 2: 5'- CGCGGCGTAACGA -3' (SEQ ID NO: 16)

Primers for analyzing SLC19A1 c.80G>A and probes

- Forward primer: 5'- GCCTGACCCCGAGCTC -3' (SEQ ID NO: 17)
- Reverse primer: 5'- CATGAAGCCGTAGAAGCAAAGGTA -3' (SEQ ID NO: 18)
- TaqMan Probe Reporter 1: 5'- ACACGAGGTGCCGCC -3' (SEQ ID NO: 19)
- TaqMan Probe Reporter 2: 5'- ACACGAGGCGCCGCC -3' (SEQ ID NO: 20)

The genomic DNA (10 ng) of each of the subjects was measured by real-time PCR assay using the TaqMan (trademark) SNP Genotyping Assays and TaqMan (trademark) GTXpress Master Mix (Life Technologies Japan Ltd.). In accordance with the protocol provided by Life Technologies Japan Ltd., specific operations were performed.

As the results of genetic polymorphism analysis on 49 subjects, multicollinearity was observed between GGH c.16T>C and GGH c.-401C>T, between SLC19A1 c.80G>A and SLC19A1 c.696T>C, and between FPGS g.130562725G>A and FPGS c.*192A>G among 8 SNPs shown in Table 3. Table 4 shows the behavior of combinations in the subjects. Table 5 shows Spearman correlation coefficients between the combinations of the SNPs.

[Table 4]

| | | GGH c.16T>C | | |
|---|---|---|---|---|
| | | T/T | T/C | C/C |
| GGH c. -401C>T | C/C | 30 | 0 | 0 |
| | C/T | 0 | 17 | 0 |
| | T/T | 0 | 0 | 2 |

| | | SLC19A1 c.80G>A | | |
|---|---|---|---|---|
| | | G/G | G/A | A/A |
| SLC19A1 c.696T>C | T/T | 0 | 0 | 10 |
| | T/C | 0 | 29 | 0 |
| | C/C | 10 | 0 | 0 |

| | | FPGS g.130562725G>A | | |
|---|---|---|---|---|
| | | G/G | G/A | A/A |
| FPGS c.*192A>G | A/A | 6 | 1 | 0 |
| | A/G | 0 | 21 | 1 |
| | G/G | 0 | 3 | 17 |

[Table 5]

| SNP1 | SNP2 | Spearman rank correlation coefficient (rs) | P value |
|---|---|---|---|
| GGH c.-401C>T | GGH c.16T>C | 1 | <.0001 |
| SLC19A1 c.696T>C | SLC19A1 c.80G>A | -1 | <.0001 |
| FPGS c.*192A>G | FPGS g. 130562725G>A | 0.8772 | <.0001 |

As shown in Table 4, the behavior of GGH c.16T>C in the subjects was completely the same as that of GGH c.-401C>T. The case of SLC19A1 c.80G>A and SLC19A1 c.696T>C was the same as described above. It was found that FPGS g.130562725G>A and FPGS c.*192A>G are not identical in behavior, but are very close to each other. As shown in Table 5, there was strong correlativity between two SNPs (SNP 1 and SNP 2) having a multicollinearity relationship with each other. Consequently, in the statistical analysis described later, either one of SNP 1 and SNP 2 of the 2 SNPs being in a multicollinearity relationship with each other may be used. In Comparative Example 1 and Examples 1 to 3 described later, FPGS c.*192 A>G, GGH c.-401C>T, and SLC19A1 c.696T>C were selected.

(4) Statistical Analysis

With respect to 5 SNPs: FPGS c.64A>G, FPGS c.*192A>G, GGH c.452C>T, GGH c.-401C>T, and SLC19A1 c.696T>C, multiple logistic analysis was performed using data of 49 subjects and a discriminant was constructed based on the regression equation (II) above (hereinafter, the constructed discriminant is referred to as "Discriminant 1"). Multiple logistic regression analysis was performed by statistical calculation software R. The "R" can be downloaded from http://www.r-project.org/. In Discriminant 1, the number of mutant alleles was treated as nominal scales (WT, HT or MT). The coefficients of Discriminant 1 are shown in Table 6. These coefficients were calculated using statistical analysis software "JMP10" (manufactured by SAS Institute Inc.). In Table 6, the space of the WT coefficient as for FPGSc.64A>G is blank. This is because 49 subjects did not fall under the requirements of the WT. Regarding the c.64 position of the FPGS gene, genotype frequency in Japanese was searched in HapMap (http://hapmap.ncbi.nlm.hih.gov/index.html.ja). The WT coefficient was 0%, the HT coefficient was 9.8%, and the MT coefficient was 90.2%.

[Table 6]

| SNPs | Coefficient in discriminant | Discriminant 1 (5SNPs) | | |
|---|---|---|---|---|
| | | WT | HT | MT |
| Piece | $b_0$ | -13.7163181884078 | | |
| FPGS c.64A>G | $b_1$ | | -8.35162705918752 | 8.35162705918752 |
| FPGS c. *192A>G | $b_2$ | 1.15276968055595 | -0.334050295162683 | -0.818719385393271 |
| GGH c. 452C>T | $b_3$ | 10.332068250275 | 11.0368605013661 | -21.3689287516412 |
| GGH c. -401C>T | $b_4$ | -4.98334781202384 | -5.51571635062725 | 10.4990641626511 |
| SLC19A1 c. 696T>C | $b_5$ | -0.469818405236261 | 0.850380477471342 | -0.380562072235081 |

ROC analysis was performed using Discriminant 1. Specifically, the efficacy of MTX in the effective and ineffective groups described in (1) was determined by Discriminant 1. When there were no mutations in all the allelic genes of the SNPs, the coefficients of the "WT" column in Table 6 were assigned to $b_1$ to $b_5$ of Discriminant 1. When there was a mutation in one of the allelic genes, the coefficients of the "HT" column were assigned thereto. When there were mutations at genetic polymorphism sites of both the allelic genes, the coefficients of the "MT" column were assigned thereto. Thus, a predictive value of efficacy was calculated. When the predictive value of efficacy was 0.5 or more, MTX was determined to be effective, whereas when the predictive value of efficacy was less than 0.5, MTX was determined to be ineffective. An ROC curve was created by plotting the X-axis as a value of "1-specificity" (i.e., false-positive rate) and plotting the Y-axis as a value of "sensitivity" (i.e., positive rate), and the area under the ROC curve (AUC) was calculated. The ROC curve was created by JMP10 (manufactured by SAS). The ROC curve is shown in Fig. 4. The sensitivity and specificity of the determination results as well as the AUC of the ROC curve in Fig. 4 are shown in Table 7 below.

[Table 7]

| | Sensitivity | Specificity | AUC |
|---|---|---|---|
| Discriminant 1 | 0.5455 | 0.8889 | 0.75673 |

(5) Determination Results

As shown in Table 7, in the case of being determined by Discriminant 1, the sensitivity is 55%, the specificity is 89%, and AUC is 0.75, and this is not a sufficiently precise determination. This suggests that it is difficult to precisely determine the efficacy of MTX only by the SNPs of the 3 genes associated with MTX metabolism. The behavior of GGH c.-401C>T in the subjects is completely the same as that of GGH c.16T>C. Thus, even if GGH c.-401C>T is replaced with GGH c.16T>C, the same determination result is assumed to be obtained. Similarly, even if SLC19A1 c.696T>C is replaced with SLC19A1 c.80G>A, the same determination result is assumed to be obtained. The behavior of FPGS c.*192A>G in the subjects is very close to that of FPGS g.130562725C>T. Accordingly, even if FPGS c.*192A>G is replaced with FPGS g.130562725G>A, the same determination result is assumed to be obtained.

Example 1

SNPs of FMO2, SLC28A3, EPHX1, and PPARG genes were further analyzed, in addition to the SNPs of FPGS, GGH, and SLC19A1 genes, and an attempt was made to determine the efficacy of MTX in patients with RA based on the analysis results.

(1) Subjects and Biological Samples

In Example 1, genomic DNA of each of the subjects obtained in Comparative Example 1 (22 subjects in the effective group and 27 subjects in the ineffective group) was used for SNP analysis.

(2) Gene Polymorphism Analysis

With respect to the genomic DNA of each of the subjects, 9 SNPs present in the 7 genes were analyzed using TaqMan (trademark) SNP Genotyping Assays (Life Technologies Japan Ltd.) and the direct sequencing method. The 9 SNPs and the methods for analyzing these SNPs are shown in Table 8. A customized product of the TaqMan (trademark) SNP Genotyping Assays was used to analyze FPGS c.64A>G.

[Table 8]

| SNP | SNP analysis method |
|---|---|
| FPGS c.64A>G | TaqMan® SNP Genotyping Assays (Assay ID: AHPAVIP) |
| FPGS c.*192A>G | TaqMan® SNP Genotyping Assays (Assay ID: C_1459148_10) |
| GGH c.452C>T | TaqMan® SNP Genotyping Assays (Assay ID: C_25623170_10) |
| GGH c.-401C>T | TaqMan® SNP Genotyping Assays (Assay ID: C_27512347_10) |
| SLC19A1 c.696T>C | TaqMan® SNP Genotyping Assays (Assay ID: C_9520092_10) |
| PPARG c.*1411G>C | TaqMan® SNP Genotyping Assays (Assay ID: C_8756542_10) |
| EPHX1 c.357G>A | TaqMan® SNP Genotyping Assays (Assay ID: C_15968483_30) |
| SLC28A3 c.267G>A | TaqMan® SNP Genotyping Assays (Assay ID: C_11764222_20) |
| FMO2 c.585A>G | Direct sequencing method |

(2-1) Analysis Based on TaqMan (trademark) SNP Genotyping Assays

The genomic DNA (10 ng) of each of the subjects was measured by real-time PCR assay using the TaqMan (trademark) SNP Genotyping Assays and TaqMan (trademark) GTXpress Master Mix (Life Technologies Japan Ltd.). In accordance with the protocol provided by Life Technologies Japan Ltd., specific operations were performed.

(2-2) Analysis by Direct Sequencing Method

The genomic DNA of each of the subjects was used as a template nucleic acid and FMO2 gene containing a genetic polymorphism site was amplified by a PCR reaction. The sequences of the forward and reverse primers used for amplification are shown below.

<FMO2 c.585A>G>

- Forward primer: 5'- TCCAGAAAGGAAAAGCTGGCAATG -3' (SEQ ID NO: 21)
- Reverse primer: 5'- GAGCCATCTCCCAGAGAAGTGAA -3' (SEQ ID NO: 22)

To the PCR reaction system, genomic DNA (2 ng/reaction) and TaKaRa Ex-Taq or TaKaRa LA-Taq (TAKARA BIO INC.) as each primer and polymerase were added. The reaction liquid obtained using TaKaRa LA-Taq as the polymerase was prepared in accordance with the protocol attached to the polymerase. The reaction liquid obtained using Ex-Taq as the polymerase was prepared by adding DMSO to the reaction liquid having the composition described in the protocol attached to the polymerase so as to have a final concentration of 5% (V/V). After the termination of reaction, the reaction liquid was purified using Ampure XP (Beckman Coulter). A part of the purified reaction liquid was subjected to agarose electrophoresis, and the presence of amplified products was confirmed. The base sequences of the amplified products were analyzed using the primer for sequencing shown below.

<FMO2 c.585A>G>

- Primer for sequencing: 5'- TGACATAGTTGCTCTGGAGC -3' (SEQ ID NO: 23)

(3) Statistical Analysis

With respect to 9 SNPs shown Table 8, multiple logistic analysis was performed using data of 49 subjects and a discriminant was constructed based on the regression equation (II) above (hereinafter, the constructed discriminant is referred to as "Discriminant 2"). Multiple logistic regression analysis was performed in the same manner as in Comparative Example 1. The coefficients of Discriminant 2 are shown in Table 9. These coefficients were calculated using JMP10 (manufactured by SAS).

[Table 9]

| SNPs | Coefficient in discriminant | Discriminant 2 (9SNPs) | | |
|---|---|---|---|---|
| | | WT | HT | MT |
| Piece | $b_0$ | -22.6684976649331 | | |
| FPGS c.64A>G | $b_1$ | | -31.0246257747404 | 31.0246257747404 |
| FPGS c.*192A>G | $b_2$ | 3.20214843740748 | -0.493945610274853 | -2.70820282713263 |
| GGH c.452C>T | $b_3$ | 0.476917228469549 | -12.4219843832566 | 11.945067154787 |
| GGH c.401C>T | $b_4$ | -3.9858463512526 | 3.15319471850915 | 0.832651632743457 |
| SLC19A1 c. 696T>C | $b_5$ | -3.26275664769377 | 0.575538353133488 | 2.68721829456028 |
| PPARG c. *1411G>C | $b_6$ | -7.47490114187799 | -11.3941468676886 | 18.8690480095666 |
| FMO2 c.585A>G | $b_7$ | 28236237479537 | -12.5156793787523 | -15.7205581007847 |
| EPHX1 c. 357G>A | $b_8$ | 11.6580555249396 | 5.76089208999637 | -17.418947614936 |
| SLC28A3 c. 267G>A | $b_9$ | -20.0551430444543 | 12.454503167113 | 7.60063987734121 |

ROC analysis was performed using Discriminant 2. Specifically, the efficacy of MTX in the effective and ineffective groups was determined by Discriminant 2 in the same manner as in Comparative Example 1. When the predictive value of efficacy was 0.5 or more, MTX was determined to be effective, whereas when the predictive value of efficacy was less than 0.5, MTX was determined to be ineffective. An ROC curve was created and the AUC was calculated. The ROC curve was created by JMP10 (manufactured by SAS). The ROC curve is shown in Fig. 5. The sensitivity and specificity of the determination results as well as the AUC of the ROC curve in Fig. 5 are shown in Table 10 below.

[Table 10]

| | Sensitivity | Specificity | AUC |
|---|---|---|---|
| Discriminant 2 | 1.0000 | 0.8519 | 0.98232 |

(5) Determination Results

As shown in Table 10, in the case of being determined by Discriminant 2, the sensitivity is 100%, the specificity is 85%, and AUC is 0.98. This showed that the precision of determination is very high. It was shown that SNPs of FMO2 gene, SLC28A3 gene, EPHX1 gene, and PPARG gene are further analyzed, in addition to the SNPs of the 3 genes associated with MTX metabolism, whereby the precision of determination in the efficacy of MTX is significantly increased. The behavior of GGH c.-401C>T in the subjects is completely the same as that of GGH c.16T>C. Thus, even if GGH c.-401C>T is replaced with GGH c.16T>C, the same determination result is assumed to be obtained. Similarly, even if SLC19A1 c.696T>C is replaced with SLC19A1 c.80G>A, the same determination result is assumed to be obtained.

Example 2

SNPs of FMO2, SLC28A3, EPHX1, and PPARG genes were analyzed, in addition to the SNPs of FPGS and GGH genes among the 3 genes associated with MTX metabolism, and an attempt was made to determine the efficacy of MTX in patients with RA based on the analysis results.

(1) Subjects and Biological Samples

In Example 2, genomic DNA of each of the subjects obtained in Comparative Example 1 (22 subjects in the effective group and 27 subjects in the ineffective group) was used for SNP analysis.

(2) Gene Polymorphism Analysis

With respect to genomic DNA of each of the subjects, 8 SNPs present in the 6 genes were analyzed in the same manner as in Example 1. The 8 SNPs are FPGS c.64A>G, FPGS c.*192A>G, GGH c.452C>T, GGH c.-401C>T, FMO2 c.585A>G, SLC28A3 c.267G>A, EPHX1 c.357G>A, andPPARG c.*1411G>C.

(3) Statistical Analysis

With respect to the 8 SNPs described above, multiple logistic analysis was performed using data of 49 subjects and a discriminant was constructed based on the regression equation (II) above (hereinafter, the constructed discriminant is referred to as "Discriminant 3"). Multiple logistic regression analysis was performed in the same manner as in Comparative Example 1. The coefficients of Discriminant 3 are shown in Table 11. These coefficients were calculated using JMP10 (manufactured by SAS).

[Table 11]

| SNPs | Coefficient in discriminant | Discriminant 3 (8SNPs) | | |
|---|---|---|---|---|
| | | WT | HT | MT |
| Piece | $b_0$ | -21.3007541838589 | | |
| FPGS c.64A>G | $b_1$ | | -29.3717696481742 | 29.3717696481742 |
| FPGS c.*192A>G | $b_2$ | 1.26196849970941 | 0.545873974218519 | -1.80784247392793 |
| GGH c.452C>T | $b_3$ | -1.02334958133483 | -8.17312010536268 | 9.19646968669751 |
| GGH c.-401C>T | $b_4$ | -1.75671850171238 | 1.5059328447617 | 0.250785656950678 |
| PPARG c. *1411G>C | $b_5$ | -6.17007011034253 | -10.8406604639507 | 17.0107305742932 |
| FMO2 c.585A>G | $b_6$ | 25.6952489774747 | -11.3390557673147 | -14.3561932101601 |
| EPHX1 c.357G>A | $b_7$ | 9.53590386709328 | 4.73003036531627 | -14.2659342324095 |
| SLC28A3 c. 267G>A | $b_8$ | -17.4636372434602 | 10.4603510624651 | 7.003286180995 |

ROC analysis was performed using Discriminant 3. Specifically, the efficacy of MTX in the effective and ineffective groups was determined by Discriminant 3 in the same manner as in Comparative Example 1. When the predictive value of efficacy was 0.5 or more, MTX was determined to be effective, whereas when the predictive value of efficacy was less than 0.5, MTX was determined to be ineffective. An ROC curve was created and the AUC was calculated. The ROC curve was created by JMP10 (manufactured by SAS). The ROC curve is shown in Fig. 6. The sensitivity and specificity of the determination results as well as the AUC of the ROC curve in Fig. 6 are shown in Table 12 below.

[Table 12]

| | Sensitivity | Specificity | AUC |
|---|---|---|---|
| Discriminant 3 | 0.9545 | 0.8889 | 0.97391 |

(5) Determination Results

As shown in Table 12, in the case of being determined by Discriminant 3, the sensitivity is 95%, the specificity is 89%, and AUC is 0.97. This showed that the precision of determination is very high. It was suggested that among the 3 genes associated with MTX metabolism, SNPs of FPGS gene and GGH gene and SNPs of FMO2 gene, SLC28A3 gene, EPHX1 gene, and PPARG gene are analyzed, whereby precise determination is possible. The behavior of GGH c.-401C>T in the subjects is completely the same as that of GGH c.16T>C. Thus, even if GGH c.-401C>T is replaced with GGH c.16T>C, the same determination result is assumed to be obtained.

Example 3

SNPs of FMO2, SLC28A3, EPHX1, and PPARG genes were analyzed, in addition to the SNP of FPGS gene among the 3 genes associated with MTX metabolism, and an attempt was made to determine the efficacy of MTX in patients with RA based on the analysis result.

(1) Subjects and Biological Samples

In Example 3, genomic DNA of each of the subjects obtained in Comparative Example 1 (22 subjects in the effective group and 27 subjects in the ineffective group) was used for SNP analysis.

(2) Gene Polymorphism Analysis

With respect to genomic DNA of each of the subjects, 6 SNPs present in the 5 genes were analyzed in the same manner as in Example 1. The 6 SNPs are FPGS c.64A>G, FPGS c.*192A>G, FMO2 c.585A>G, SLC28A3 c.267G>A, EPHX1 c.357G>A, and PPARG c.*1411G>C.

(3) Statistical Analysis

With respect to the 6 SNPs described above, multiple logistic analysis was performed using data of 49 subjects and a discriminant was constructed based on the regression equation (II) above (hereinafter, the constructed discriminant is referred to as "Discriminant 4"). Multiple logistic regression analysis was performed in the same manner as in Comparative Example 1. The coefficients of Discriminant 4 are shown in Table 13. These coefficients were calculated using JMP10 (manufactured by SAS).

[Table 13]

| SNPs | Coefficient in Discriminant | Discriminant 4 (6SNPs) | | |
|---|---|---|---|---|
| | | WT | HT | MT |
| Piece | $b_0$ | -20.4847300244848 | | |
| FPGS c.64A>G | $b_1$ | | -26.1044904071444 | 26.1044904071444 |
| FPGS c.*192A>G | $b_2$ | 0.364068615783232 | 1.0872716968462 | -1.45134031262943 |
| PPARG c.*1411G>C | $b_3$ | -5.29127211104476 | -8.96595444112521 | 14.25722655217 |
| FMO2 c.585A>G | $b_4$ | 21.8984585660587 | -10.0894028958972 | -11.8090556701614 |
| EPHX1 c.357G>A | $b_5$ | 6.68953572295613 | 4.32564941302424 | -11.0151851359804 |

(continued)

| SNPs | Coefficient in Discriminant | Discriminant 4 (6SNPs) | | |
|---|---|---|---|---|
| | | WT | HT | MT |
| SLC28A3 c.267G>A | $b_6$ | -13.4260945963955 | 7.36957966251219 | 6.05651493388332 |

ROC analysis was performed using Discriminant 4. Specifically, the efficacy of MTX in the effective and ineffective groups was determined by Discriminant 4 in the same manner as in Comparative Example 1. When the predictive value of efficacy was 0.5 or more, MTX was determined to be effective, whereas when the predictive value of efficacy was less than 0.5, MTX was determined to be ineffective. An ROC curve was created and the AUC was calculated. The ROC curve was created by JMP10 (manufactured by SAS). The ROC curve is shown in Fig. 7. The sensitivity and specificity of the determination results as well as the AUC of the ROC curve in Fig. 7 are shown in Table 14 below.

[Table 14]

| | Sensitivity | Specificity | AUC |
|---|---|---|---|
| Discriminant 4 | 0.9091 | 0.8889 | 0.95791 |

(5) Determination Results

As shown in Table 14, in the case of being determined by Discriminant 4, the sensitivity is 91%, the specificity is 89%, and AUC is 0.95. This showed that the precision of determination is very high. It was suggested that among the 3 genes associated with MTX metabolism, SNP of FPGS gene and SNPs of FMO2 gene, SLC28A3 gene, EPHX1 gene, and PPARG gene are analyzed, whereby precise determination is possible.

Example 4

Analysis was performed using FPGS g.130562725G>A in place of FPGS c.*192A>G among the SNPs used in Examples 1 to 3, and an attempt was made to determine the efficacy of MTX in patients with RA based on the analysis results.

(1) Subjects and Biological Samples

In Example 4, genomic DNA of each of the subjects obtained in Comparative Example 1 (22 subjects in the effective group and 27 subjects in the ineffective group) was used for SNP analysis.

(2) Gene Polymorphism Analysis

With respect to genomic DNA of each of the subjects, FPGS g.130562725G>A was analyzed in the same manner as in Comparative Example 1. FPGS c.64A>G, GGH c.452C>T, GGH c.-401C>T, FMO2 c.585A>G, SLC28A3 c.267G>A, EPHX1 c.357G>A, and PPARG c.*1411G>C were analyzed in the same manner as in Example 1.

(3) Statistical Analysis

In Example 4, the combinations of SNPs shown in Table 15 below were used. In Table 15, analysis data of SNPs marked with "●" were used for the discriminants described later.

[Table 15]

| SNP | Discriminant 5 (9 SNPs) | Discriminant 6 (8 SNPs) | Discriminant 7 (6 SNPs) |
|---|---|---|---|
| FPGS c.64A>G | ● | ● | ● |
| FPGS g.130562725G>A | ● | ● | ● |
| GGH c.452C>T | ● | ● | |
| GGH c.-401C>T | ● | ● | |

(continued)

| SNP | Discriminant 5 (9 SNPs) | Discriminant 6 (8 SNPs) | Discriminant 7 (6 SNPs) |
|---|---|---|---|
| SLC19A1 c.696T>C | ● | | |
| FMO2 c.585A>G | ● | ● | ● |
| SLC28A3 c.267G>A | ● | ● | ● |
| EPHX1 c.357G>A | ● | ● | ● |
| PPARG c.*1411G>C | ● | ● | ● |

With respect to the SNPs shown in Table 15, multiple logistic analysis was performed using data of 49 subjects and a discriminant was constructed based on the regression equation (II) above (hereinafter, the constructed discriminants are referred to as "Discriminant 5", "Discriminant 6", and "Discriminant 7", respectively). Multiple logistic regression analysis was performed in the same manner as in Comparative Example 1. The coefficients of the respective discriminants are shown in Tables 16 to 18. These coefficients were calculated using JMP10 (manufactured by SAS).

[Table 16]

| SNPs | Coefficient in Discriminant | Discriminant 5 (9SNPs) | | |
|---|---|---|---|---|
| | | WT | HT | MT |
| Piece | $b_0$ | -23.3712864094754 | | |
| FPGS c.64A>G | $b_1$ | | -30.9209463104518 | 30.9209463104518 |
| FPGS g.130562725G>A | $b_2$ | 3.12127373881671 | -0.444151451828988 | -2.67712228698772 |
| GGH c.452C>T | $b_3$ | 1.135197486140251 | -11.5168459927843 | 10.3808711313818 |
| GGH c.-401C>T | $b_4$ | -3.87985088016462 | 3.15101724463755 | 0.728833635527066 |
| SLC19A1 c.696T>C | $b_5$ | -3.18975795520665 | 0.56244526442843 | 2.62731269077822 |
| PPARG c.*1411G>C | $b_6$ | -7.4922581208362 | -11.3464951073154 | 18.8387532281516 |
| FMO2 c.585A>G | $b_7$ | 28.0763260887622 | -12.4183439248173 | -15.6579821639449 |
| EPHX1 c.357G>A | $b_8$ | 11.5467660437045 | 5.75537831826951 | -17.302144361974 |
| SLC28A3 c.267G>A | $b_9$ | -19.971092546653 | 12.3801366302726 | 7.59095591638042 |

[Table 17]

| SNPs | Coefficient in Discriminant | Discriminant 6 (8SNPs) | | |
|---|---|---|---|---|
| | | WT | HT | MT |
| Piece | $b_0$ | -22.0717858681062 | | |
| FPGS c.64A>G | $b_1$ | | -29.2901492892033 | 29.2901492892033 |
| FPGS g.130562725G>A | $b_2$ | 1.20926200597196 | 0.577924858285745 | -1.7871868642577 |
| GGH c.452C>T | $b_3$ | -0.287204534599967 | -7.22840958805964 | 7.51561412265961 |
| GGH c.-401C>T | $b_4$ | -1.67996690545532 | 1.52779558221935 | 0.152171323235968 |
| PPARG c.*1411G>C | $b_5$ | -6.22143096287302 | -10.771185021489 | 16.992615984362 |
| FMO2 c.585A>G | $b_6$ | 25.5875414947055 | -11.2795950281185 | -14.3079464665869 |
| EPHX1 c.357G>A | $b_7$ | 9.43995710754343 | 4.75538234555698 | -14.1953394531004 |
| SLC28A3 c.267G>A | $b_8$ | -17.3715707973361 | 10.3795531574804 | 6.99201763985576 |

[Table 18]

| SNPs | Coefficient in discriminant | Discriminant 7 (6SNPs) | | |
|---|---|---|---|---|
| | | WT | HT | MT |
| Piece | $b_0$ | -20.5254450961768 | | |
| FPGS c. 64A>G | $b_1$ | | -26.0833438083665 | 26.0833438083665 |
| FPGS g. 130562725G>A | $b_2$ | 0.366164884559452 | 1.13837912955029 | -1.50454401410974 |
| PPARG c.* 1411G>C | $b_3$ | -5.42922193000107 | -8.86811253461943 | 14.2973344646205 |
| FMO2 c.585A>G | $b_4$ | 21.9330126407613 | -10.0235095750505 | -11.9095030657109 |
| EPHX1 c.357G>A | $b_5$ | 6.6483497129094 | 4.43600840737558 | -11.084358120285 |
| SLC28A3 c.267G>A | $b_6$ | -13.359926506358 | 7.30144128004176 | 6.05848522631628 |

ROC analysis was performed using each of Discriminants 5 to 7. Specifically, the efficacy of MTX in the effective and ineffective groups was determined by each of Discriminants in the same manner as in Comparative Example 1. When the predictive value of efficacy was 0.5 or more, MTX was determined to be effective, whereas when the predictive value of efficacy was less than 0.5, MTX was determined to be ineffective. An ROC curve was created and the AUC was calculated. The ROC curve was created by JMP10 (manufactured by SAS). The ROC curves of Discriminants 5 to 7 are shown in Figs. 8 to 10. The sensitivity and specificity of the determination results as well as the AUC of the ROC curve are shown in Table 19 below.

[Table 19]

| | Sensitivity | Specificity | AUC |
|---|---|---|---|
| Discriminant 5 | 1.0000 | 0.8519 | 0.98232 |
| Discriminant 6 | 0.9545 | 0.8889 | 0.97391 |
| Discriminant 7 | 0.9091 | 0.8889 | 0.96128 |

As shown in Table 19, it was found that even if any of Discriminants 5 to 7 is used, the precision of determination is very high. Therefore, even if analysis is performed using FPGS g.130562725G>A in place of FPGS c.*192A>G among the SNPs used in Examples 1 to 3, it is possible to precisely determine the efficacy of MTX in patients with RA.

## Claims

**1.** A method for assisting diagnosis of efficacy of methotrexate (MTX) in a patient with rheumatoid arthritis, the method comprising the steps of:

analyzing in a biological sample obtained from a patient with rheumatoid arthritis:

- the single nucleotide polymorphism (SNP) of the FPGS gene FPGS c.64A>G and either one of FPGS c.*192A>G and rs1544105;
- the SNP of the FMO2 gene FMO2 c.585A>G,
- the SNP of the SLC28A3 gene SLC28A3 c.267G>A,
- the SNP of the EPHX1 gene EPHX1 c.357G>A; and
- the SNP of the PPARG gene PPARG c.*1411G>C; and

obtaining a numerical index associated with the efficacy of MTX from the analysis result,
comparing the obtained numerical index with a predetermined threshold, and
determining the efficacy of MTX in the patient based on the comparison results.

**2.** The method according to claim 1, wherein in the analysis step, the SNPs of the GGH gene GGH c.452C>T, and either one of GGH c.-401C>T, or GGH c.16T>C, are further analyzed.

**3.** The method according to claim 2, wherein in the analysis step, one SNP of the SLC19A1 gene selected from SLC19A1 c.696T>C and SLC19A1 c.80G>A is further analyzed.

**4.** The method according to any one of claims 1 to 3, wherein the numerical index associated with the efficacy of MTX is a predictive value calculated by a multiple logistic model.

**5.** The method according to claim 4, wherein when the predictive value is more than or equal to the predetermined threshold in the determining step, the efficacy of MTX in the patient is determined to be high.

**6.** The method according to claim 4, wherein when the predictive value is lower than the predetermined threshold, the efficacy of MTX in the patient is determined to be low.

**7.** A device for determining efficacy of methotrexate in a patient with rheumatoid arthritis, comprising a computer, wherein the computer comprises a processor and a memory under control of the processor,
wherein the memory stores a computer program that allows the device to execute the steps of:

analyzing in a biological sample obtained from a patient with rheumatoid arthritis:

- the single nucleotide polymorphism (SNP) of the FPGS gene FPGS c.64A>G and either one of FPGS c.*192A>G and rs1544105,
- the SNP of the FMO2 gene FMO2 c.585A>G,
- the SNP of the SLC28A3 gene SLC28A3 c.267G>A,
- the SNP of the EPHX1 gene EPHX1 c.357G>A, and
- the SNP of the PPARG gene PPARG c.*1411G>C; and

obtaining a numerical index associated with the efficacy of MTX from the analysis result,
comparing the obtained numerical index with a predetermined threshold, and
determining the efficacy of MTX in the patient based on the comparison results.

**8.** The device according to claim 7, wherein in the analysis step, the SNPs of the GGH gene GGH c.452C>T, and either one of GGH c.-401C>T, or GGH c.16T>C, are further analyzed.

**9.** The device according to claim 8, wherein in the analysis step, one SNP of the SLC19A1 gene selected from SLC19A1 c.696T>C and SLC19A1 c.80G>A is further analyzed.

## Patentansprüche

**1.** Verfahren zur Unterstützung der Diagnose der Wirksamkeit von Methotrexat (MTX) bei einem Patienten mit rheumatoider Arthritis, wobei das Verfahren die Schritte umfasst:

Analysieren in einer biologischen Probe, die von einem Patienten mit rheumatoider Arthritis erhalten wird: - des Einzelnukleotidpolymorphismus (SNP) des FPGS-Gens FPGS c.64A>G und eines von FPGS c.*192A>G und rs1544105;

- des SNP des FMO2-Gens FM02 c.585A>G,
- des SNP des SLC28A3-Gens SLC28A3 c.267G>A,
- des SNP des EPHX1-Gens EPHX1 c.357G>A; und
- des SNP des PPARG-Gens PPARG c.*1411G>C; und

Erfassen eines numerischen Index, der mit der Wirksamkeit von MTX assoziiert ist, aus dem Analyseergebnis,
Vergleichen des numerischen Index mit einer festgelegten Schwelle, und
Bestimmen der Wirksamkeit von MTX in dem Patienten auf der Basis der Vergleichsergebnisse.

**2.** Verfahren nach Anspruch 1, wobei in dem Analyseschritt die SNPs des GGH-Gens GGH c.452C>T und eines von GGH c.-401C>T oder GGH c.16T>C weiter analysiert werden.

**3.** Verfahren nach Anspruch 2, wobei in dem Analyseschritt ein SNP des SLC19A1-Gens, ausgewählt aus SLC19A1 c.696T>C und SCL19A1 c.80G>A, weiter analysiert wird.

**4.** Verfahren nach einem der Ansprüche 1 bis 3, wobei der numerische Index, der mit der Wirksamkeit von MTX assoziiert ist, ein Vorhersagewert ist, der durch ein multiples logistisches Modell berechnet wird.

**5.** Verfahren nach Anspruch 4, wobei, wenn der Vorhersagewert in dem Bestimmungsschritt größer oder gleich dem festgelegten Schwellenwert ist, die Wirksamkeit von MTX bei dem Patienten als hoch bestimmt wird.

**6.** Verfahren nach Anspruch 4, wobei, wenn der Vorhersagewert niedriger als der festgelegte Schwellenwert ist, die Wirksamkeit von MTX bei dem Patienten als niedrig bestimmt wird.

**7.** Vorrichtung zur Bestimmung der Wirksamkeit von Methotrexat bei einem Patienten mit rheumatoider Arthritis, umfassend einen Computer,
wobei der Computer einen Prozessor und einen Speicher unter der Steuerung des Prozessors umfasst,
wobei der Speicher ein Computerprogramm speichert, das es der Vorrichtung ermöglicht, die Schritte auszuführen:

Analysieren in einer biologischen Probe, die von einem Patienten mit rheumatoider Arthritis erhalten wird:

- des Einzelnukleotidpolymorphismus (SNP) des FPGS-Gens FPGS c.64A>G und eines von FPGS c.*192A>G und rs1544105;
- des SNP des FMO2-Gens FM02 c.585A>G,
- des SNP des SLC28A3-Gens SLC28A3 c.267G>A,
- des SNP des EPHX1-Gens EPHX1 c.357G>A; und
- des SNP des PPARG-Gens PPARG c.*1411G>C; und

Erfassen eines numerischen Index, der mit der Wirksamkeit von MTX assoziiert ist, aus dem Analyseergebnis,
Vergleichen des numerischen Index mit einer festgelegten Schwelle, und
Bestimmen der Wirksamkeit von MTX in dem Patienten auf der Basis der Vergleichsergebnisse.

**8.** Vorrichtung nach Anspruch 7, wobei in dem Analyseschritt die SNPs des GGH-Gens GGH c.452C>T und eines von GGH c.-401C>T oder GGH c.16T>C weiter analysiert werden.

**9.** Vorrichtung nach Anspruch 8, wobei in dem Analyseschritt ein SNP des SLC19A1-Gens, ausgewählt aus SLC19A1 c.696T>C und SLC19A1 c.80G>A, weiter analysiert wird.

## Revendications

**1.** Méthode d'aide au diagnostic de l'efficacité du méthotrexate (MTX) chez un patient souffrant de polyarthrite rhumatoïde, la méthode comprenant les étapes consistant à :

analyser dans un échantillon biologique obtenu auprès d'un patient souffrant de polyarthrite rhumatoïde :

- le polymorphisme nucléotidique (SNP) du gène FPGS, FPGS c.64A>G et l'un ou l'autre des membres du groupe constitué par FPGS c.*192A>G et rs1544105 ;
- le SNP du gène FM02, FM02 c.585A>G,
- le SNP du gène SLC28A3, SLC28A3 c.267G>A,
- le SNP du gène EPHX1, EPHX1 c.357G>A ; et
- le SNP du gène PPARG, PPARG c.*1411G>C ; et

obtenir un indice numérique associé à l'efficacité du MTX à partir du résultat d'analyse,
comparer l'indice numérique obtenu à un seuil prédéterminé, et
déterminer l'efficacité du MTX chez le patient en se basant sur les résultats de comparaison.

**2.** Méthode selon la revendication 1, où dans l'étape d'analyse, les SNP du gène GGH, GGH c.452C>T, et l'un ou l'autre des membres du groupe constitué par GGH c.-401C>T ou GGH c.16T>C, sont analysés plus avant.

3. Méthode selon la revendication 2, où dans l'étape d'analyse, un SNP du gène SLC19A1 choisi parmi SLC19A1 c.696T>C et SLC19A1 c.80G>A est analysé plus avant.

4. Méthode selon l'une quelconque des revendications 1 à 3, où l'indice numérique associé à l'efficacité du MTX est une valeur prédictive calculée par un modèle logique multiple.

5. Méthode selon la revendication 4, où lorsque la valeur prédictive est supérieure ou égale au seuil prédéterminé dans l'étape de détermination, l'efficacité du MTX chez le patient est déterminée comme étant élevée.

6. Méthode selon la revendication 4, où lorsque la valeur prédictive est inférieure au seuil prédéterminé, l'efficacité du MTX chez le patient est déterminée comme étant basse.

7. Dispositif de détermination de l'efficacité du méthotrexate chez un patient souffrant de polyarthrite rhumatoïde, comprenant un ordinateur,
où l'ordinateur comprend un processeur et une mémoire sous le contrôle du processeur,
où la mémoire stocke un programme informatique qui permet au dispositif d'exécuter les étapes consistant à :

analyser dans un échantillon biologique obtenu auprès d'un patient souffrant de polyarthrite rhumatoïde :

- le polymorphisme nucléotidique (SNP) du gène FPGS, FPGS c.64A>G et l'un ou l'autre des membres du groupe constitué par FPGS c.*192A>G et rs1544105,
- le SNP du gène FM02, FM02 c.585A>G,
- le SNP du gène SLC28A3, SLC28A3 c.267G>A,
- le SNP du gène EPHX1, EPHX1 c.357G>A, et
- le SNP du gène PPARG, PPARG c.*1411G>C ; et

obtenir un indice numérique associé à l'efficacité du MTX à partir du résultat d'analyse,
comparer l'indice numérique obtenu à un seuil prédéterminé, et
déterminer l'efficacité du MTX chez le patient en se basant sur les résultats de comparaison.

8. Dispositif selon la revendication 7, où dans l'étape d'analyse, les SNP du gène GGH, GGH c.452C>T, et l'un ou l'autre des membres du groupe constitué par GGH c.-401C>T ou GGH c.16T>C, sont analysés plus avant.

9. Dispositif selon la revendication 8, où dans l'étape d'analyse, un SNP du gène SLC19A1 choisi parmi SLC19A1 c.696T>C et SLC19A1 c.80G>A est analysé plus avant.

FIG. 1

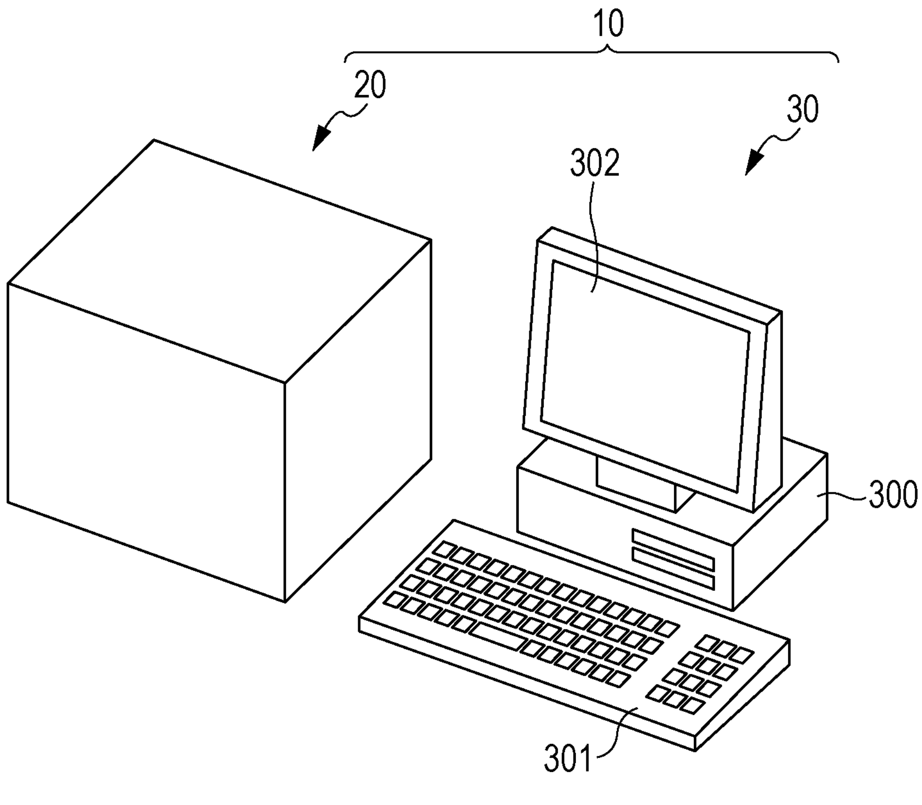

FIG. 2

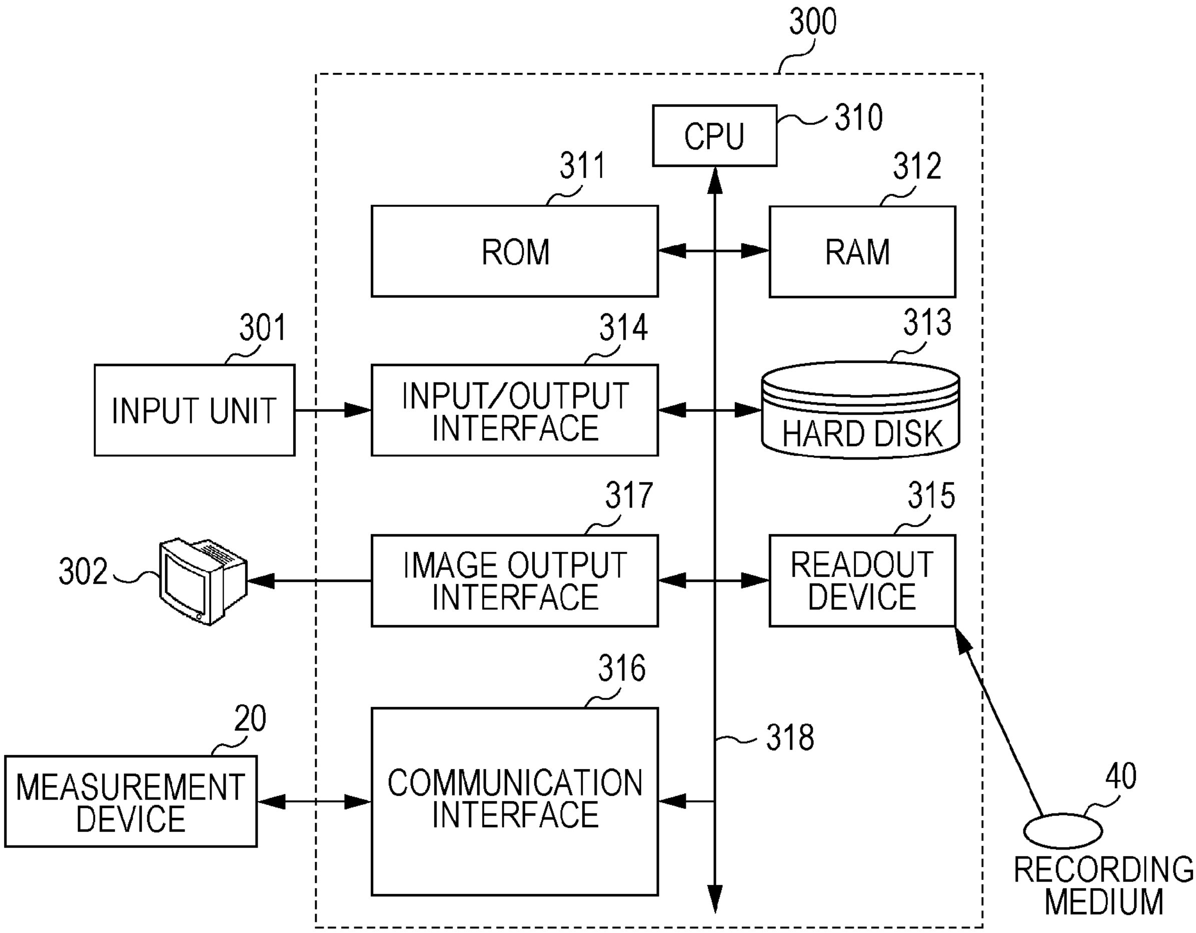
300
CPU
310
311
312
ROM
RAM
301
314
313
INPUT UNIT
INPUT/OUTPUT INTERFACE
HARD DISK
317
315
302
IMAGE OUTPUT INTERFACE
READOUT DEVICE
316
20
318
MEASUREMENT DEVICE
COMMUNICATION INTERFACE
40
RECORDING MEDIUM

FIG. 3

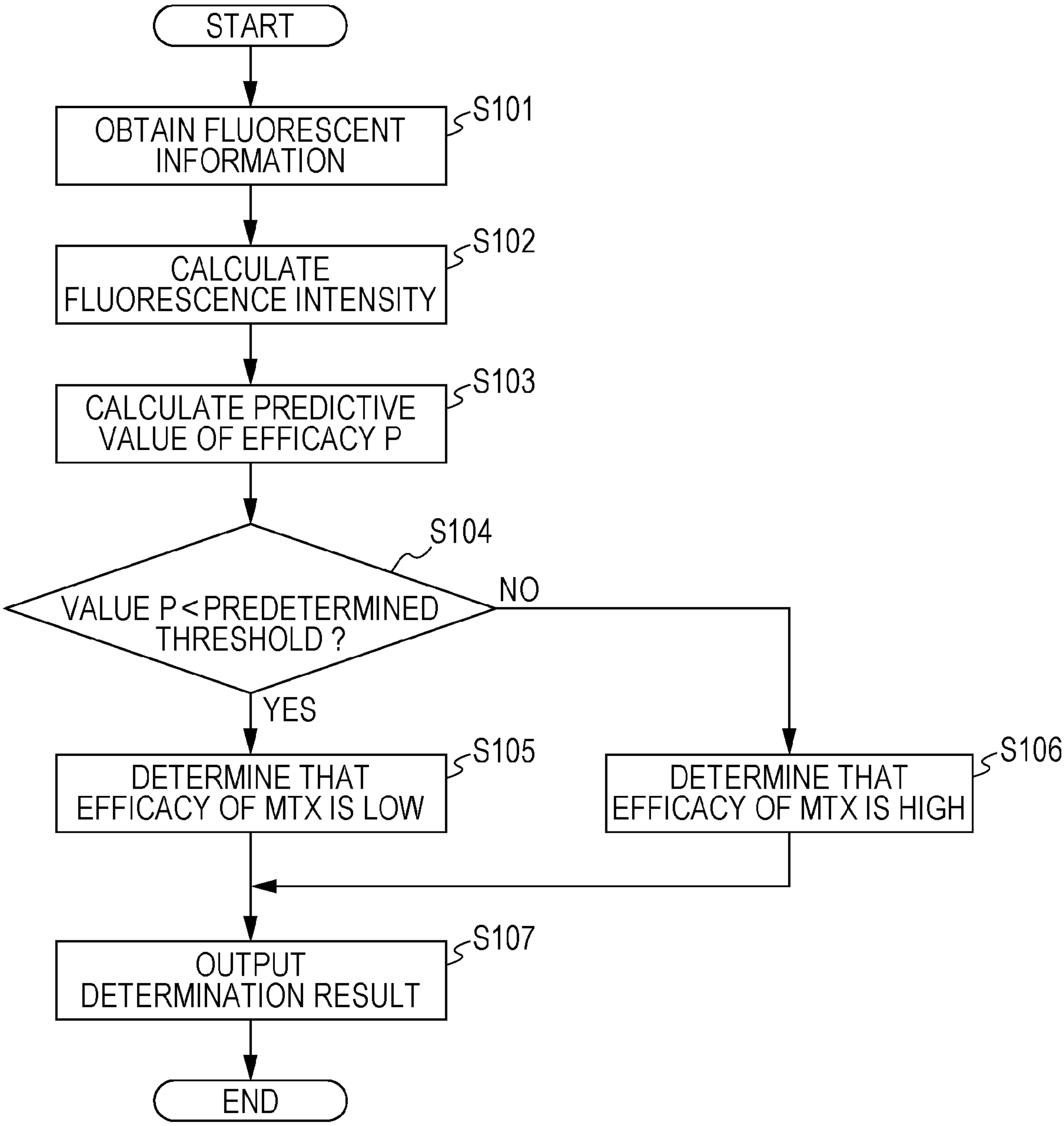
START
OBTAIN FLUORESCENT INFORMATION
S101
CALCULATE FLUORESCENCE INTENSITY
S102
CALCULATE PREDICTIVE VALUE OF EFFICACY P
S103
VALUE P < PREDETERMINED THRESHOLD ?
S104
NO
YES
DETERMINE THAT EFFICACY OF MTX IS LOW
S105
DETERMINE THAT EFFICACY OF MTX IS HIGH
S106
OUTPUT DETERMINATION RESULT
S107
END

FIG. 4

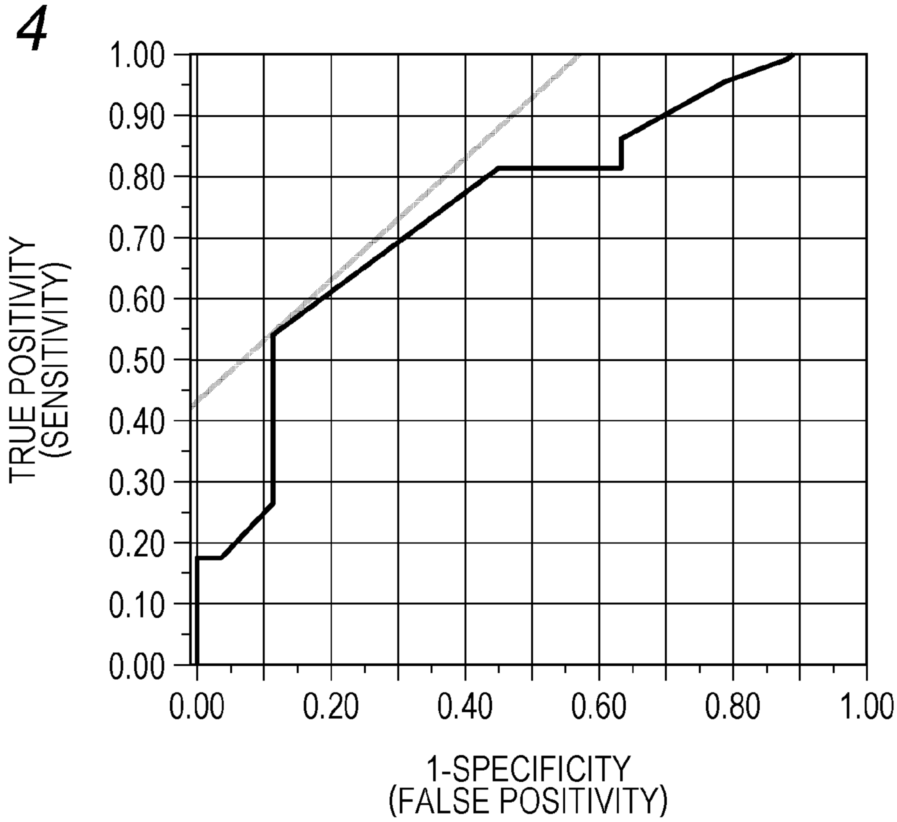
1.00
0.90
0.80
0.70
0.60
0.50
0.40
0.30
0.20
0.10
0.00
TRUE POSITIVITY
(SENSITIVITY)
0.00
0.20
0.40
0.60
0.80
1.00
1-SPECIFICITY
(FALSE POSITIVITY)

FIG. 5

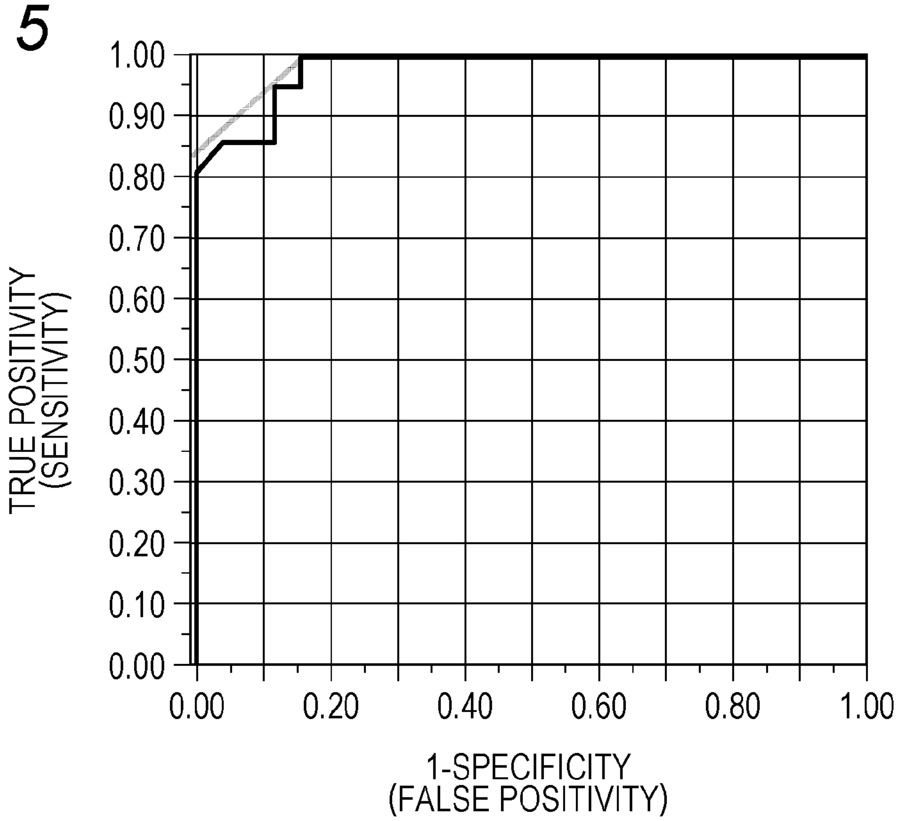
1.00
0.90
0.80
0.70
0.60
0.50
0.40
0.30
0.20
0.10
0.00
TRUE POSITIVITY
(SENSITIVITY)
0.00
0.20
0.40
0.60
0.80
1.00
1-SPECIFICITY
(FALSE POSITIVITY)

FIG. 6

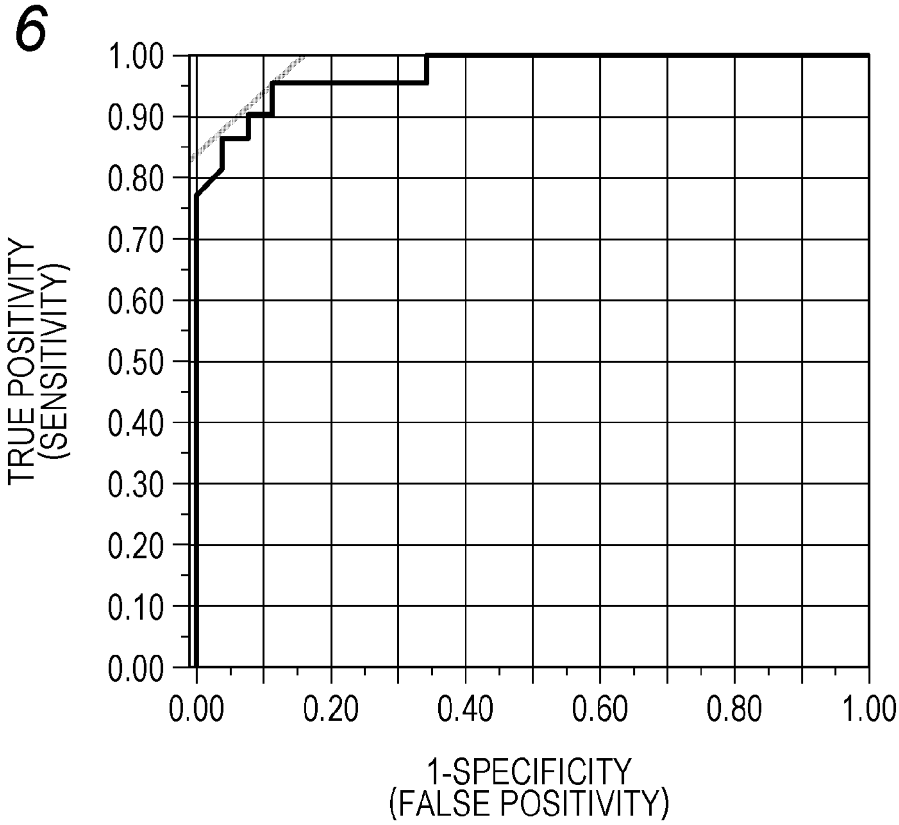
1.00
0.90
0.80
0.70
0.60
0.50
0.40
0.30
0.20
0.10
0.00
TRUE POSITIVITY
(SENSITIVITY)
0.00
0.20
0.40
0.60
0.80
1.00
1-SPECIFICITY
(FALSE POSITIVITY)

FIG. 7

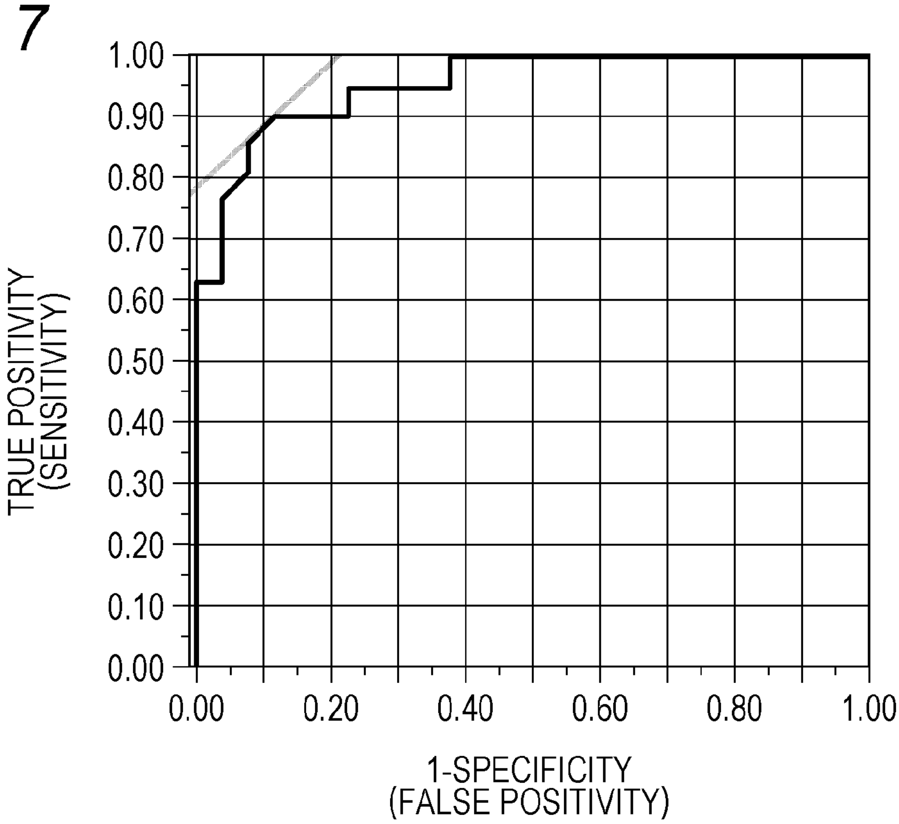
1.00
0.90
0.80
0.70
0.60
0.50
0.40
0.30
0.20
0.10
0.00
TRUE POSITIVITY
(SENSITIVITY)
0.00
0.20
0.40
0.60
0.80
1.00
1-SPECIFICITY
(FALSE POSITIVITY)

FIG. 8

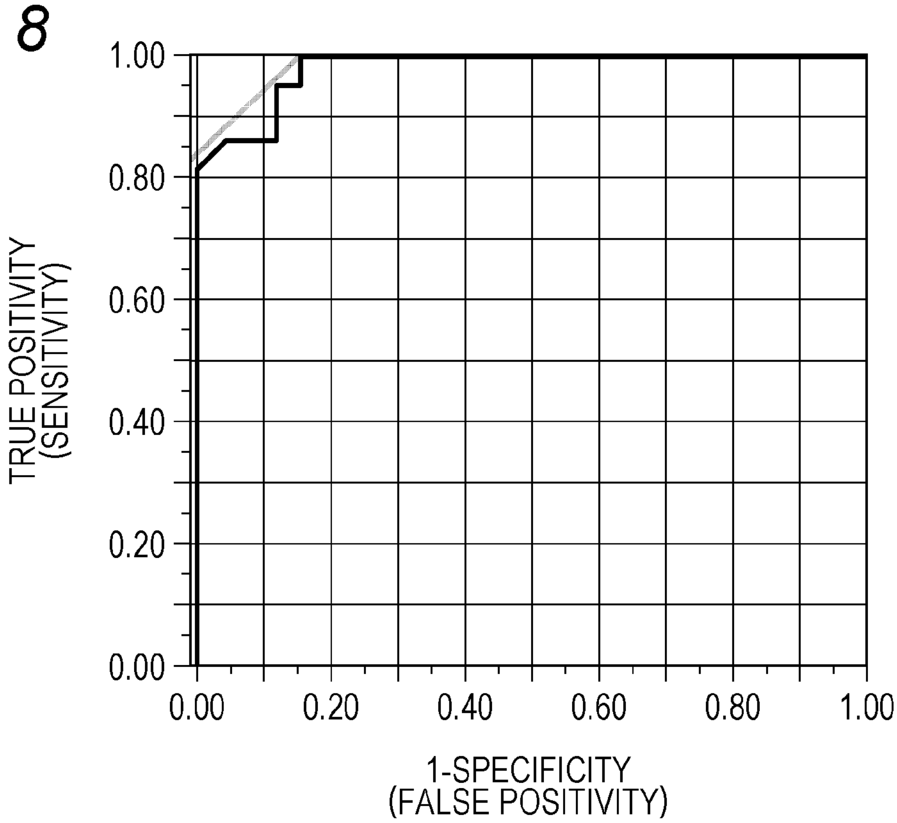
1.00
0.80
0.60
0.40
0.20
0.00
TRUE POSITIVITY (SENSITIVITY)
0.00
0.20
0.40
0.60
0.80
1.00
1-SPECIFICITY (FALSE POSITIVITY)

FIG. 9

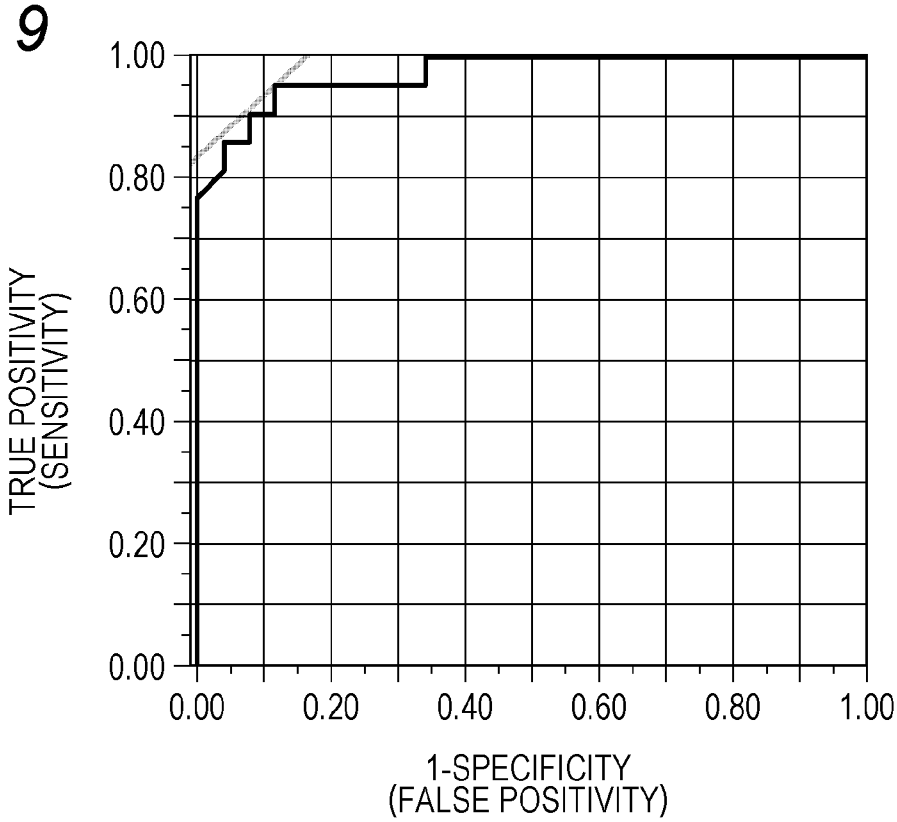
1.00
0.80
0.60
0.40
0.20
0.00
TRUE POSITIVITY (SENSITIVITY)
0.00
0.20
0.40
0.60
0.80
1.00
1-SPECIFICITY (FALSE POSITIVITY)

FIG. 10

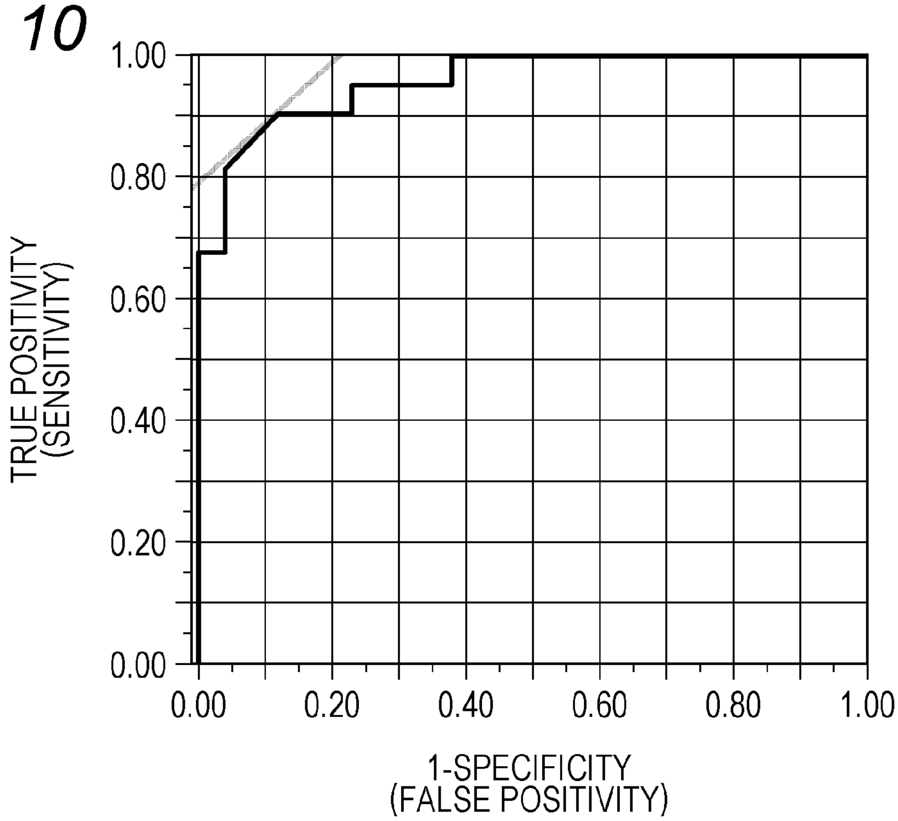
1.00
0.80
0.60
0.40
0.20
0.00
TRUE POSITIVITY
(SENSITIVITY)
0.00
0.20
0.40
0.60
0.80
1.00
1-SPECIFICITY
(FALSE POSITIVITY)

FIG. 11

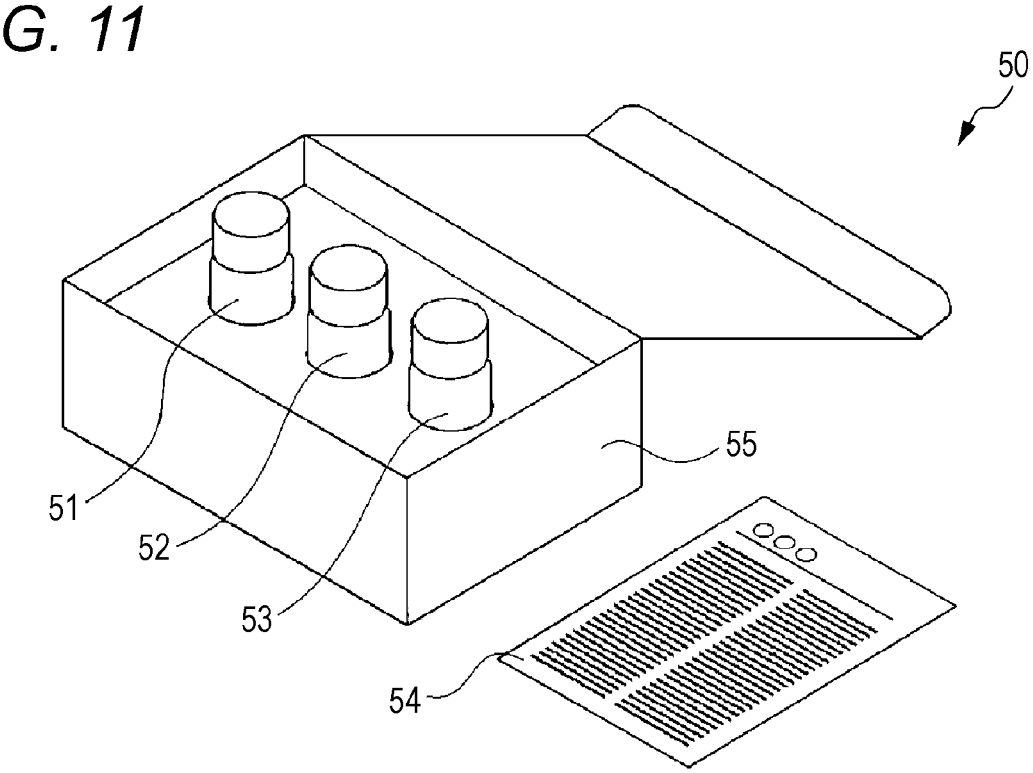
50
51
52
53
54
55

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4950414 B
- JP 2015114056 A


### Non-patent literature cited in the description


- **DAVILA L. ; RANGANATHAN P.** *Nat. Rev. Rheumatol,* 2011, vol. 7, 537-550
- **WESSELS J. A. M. et al.** *Arthritis Rheum,* 2007, vol. 56, 1765-1775
- **HAYASHI H. et al.** *J. Clin. Pharmacy Therapeutics,* 2009, vol. 34, 355-361

SEQUENCE LISTING

<110> SYSMEX CORPORATION SHINKO MEDICAL CORPORATION

<120> METHOD AND DEVICE FOR ASSISTING DIAGNOSIS OF EFFICACY OF METHOTREXATE IN PATIENT WITH RHEUMATOID ARTHRITIS

<130> SYSM-109-EP

<150> JP2015-114056
<151> 2015-06-04

<160> 23

<170> PatentIn version 3.5

<210> 1
<211> 1001
<212> DNA

<213> Homo sapiens

<400> 1

```
ttctctaaac ggcagcagct gtttagaggt gtccaccagg tgtccgcagc tttgtcatcc      60
tatccctgtt cggggcagag actgagggct gctgacccgg accggctatt ttgggacgtg     120
ctgcgggggg ccttgggagg ttggtgacga aaggagtgcg tgcccgctaa gggaggggac     180
gccccggagc gtacactcat aaacctggtc ccgaggcctg cccctcacca ggatggtgca     240
cgcggaaggg gcggcttttt agtggcgcaa gggggctggt cggtggtagt ttggggcggt     300
gctgattgat ggcgggcggg gcggggcggt gctgattggc gggggggcg gggtgaggcg      360
acgctgcgct gattggctgg gggcggggcg gggcgtctcc cgcccgggcc tagagcgctg     420
ccgggggcgc cgggactatg tcgcgggcgc ggagccacct gcgcgccgct ctattcctgg     480
cagcggcgtc tgcgcgcggc ataacgaccc aggtcgcggc gcggcggggc ttgagcgcgt     540
ggccggtgcc gcaggagccg agcatggagt accaggtatc aggcgggcca gcgggccagc     600
gggcctgggc gcgacgacac gtgggcctgc gctgagccgc agaacatccg ggctccgcta     660
gccgagaggg tatcgggagc cctggactgg gggactcggg gggcggaaca tcctggaggc     720
tgggggtggg gacagggacc aggaagttgg gcccgggccg ccggggctgg gaattcggag     780
actatagcgt ccccgccccg ggttgggaag tgggaagtgg cacaggagct aggatccaga     840
agcccagagg ctcagcggtg cttctggagt tccagtgatc ccggagtctg aaccggcagt     900
gagagtgggg aaagagggta gggaagagac tcaggaattc aggcttgaaa gatccaggag     960
tattgatctg gggtgggct gtccaggatt cagaagattg g                         1001
```

<210> 2
<211> 1001
<212> DNA
<213> Homo sapiens

<400> 2

```
tctctgcaag gggtctgca gaaagcactg cctggcatat ggtgaacagg cttcagtctg        60
gtcttcttct catctcacag acaggccacc tgaggcgcac agggagggag ggacttgttc      120
caggccacac agccaggctc cctctacaca gtgcaggtcc ctgaccacca gcagcccctc      180
cctccgtctc tgctgaggga gcagggggtc aagggtcagt cgaaggcttt gccatttctt      240
taaaaaacaa aggctttaat tcactccagc caccctctgt cccgcagtgt cccaggcaga      300
ggccctctcc cttcccagaa ggcactggga ggaggaaatc taccacccag cacatggcaa      360
gacccagggc ctggccaggc agcgcacaca ataagctggg cctgagccag gaggcaggcg      420
gggtgttggg ggagacaggc tgaacggcca ggccactgca acagtgagag acccggggag      480
cccctctgct atctcagcca agaggaagga gagaccaagg cacagagcct taaaaaagac      540
atcctctccc ggcctcccat cccaaagccc tggcccctag gccagtctag acagaaccag      600
gaaagccaaa aacaaaaggc acctagtatg taagttcatg gggagaacgc aggcaggtgt      660
gggaagctcc cacctccaac cccgggcctt ggctactggg acagtgcggg ctccagcagc      720
ttcaggacac cacccaccag gtgcaggctg ccagtgacta gcacatggat ggcagcagcc      780
tcacggagta tgctggcccc actgtgagcc acagggtggg tgaggaggcc ctttggggga      840
ctaggtggct ggaagatggg gtctcggcct tggctgatcc attgcaaggc atgtgaaatg      900
cagctgaaga cgagggagct ggcactgcag gtgtggggtg ggtggggcgc cagaagcagg      960
gatgcggacc caccgggctc tgggctgggg gcactccaga g                          1001
```

<210> 3
<211> 1001
<212> DNA
<213> Homo sapiens

<400> 3

```
gtgccatcac acccggctaa tttttgtatt tttagtagag acggggtttc gccatgttgg        60
ccagtctggt cttgaacccc tgacctcagg tgatccacct tcctcggcct cccaaagtgc       120
tgggattaca tgcttgagcc accacgccca gcccttcgca aacactttaa gtagtacctt       180
tctagattca gacactgcta taaacacttc tgacacagtt gctcatttaa tcttcatcac       240
aaacccatga aataggtact aatattatca tataggccca aaggagtaaa agcatatgct       300
caagtcacaa aaccaagatt tgacgtctgg ccccagagtc cttattctta gcctctgcgc       360
ctccttccct gtctccccag ggacactgct aattcagcgt ctttaatggc cgtatggcct       420
tcccaggagc atcctggatt cgcctgtgga agggcattca ttctgccttc agtgttttgc       480
tgctatggat atgcgtttac gtcgaagggc ttgatttctg ggagttggat tgcatggagg       540
ggtggggttg ggtccaggga tgtttgcaat ccttttcccc aatgctgctg tagtcttggc       600
atcccagcag ctgtgtgtga aggaggcacc cctctcccag gatgccccca gaccacaggg       660
tgcagagaac catctcagtg tttctgtcat tcccctgctg cagtgaactt gagtatcttc       720
ccatgcattt atgtcagtga actgcctaac ccatccttgg cccatttttc taatgtctga       780
ttcgcttttt catgtcagtt ataatagccc tttgtgggct gggtgcagtg gctcacacct       840
gtaatcctag cactttggaa gactgaggcg ggaagattgc ttgaagccta gagttctaga       900
tcagcctggg caacatggtg aaaccccctt ctctacaaaa agcaaaaatt gctgggcatg       960
ctggtgcaca tctgtggtcc cagtgatttg gaggctgagg c                          1001
```

<210> 4
<211> 1001
<212> DNA
<213> Homo sapiens

<400> 4

```
taccatagct caaacctttg ctgtttctct cctcaactat taacagtgat ttttaaatta        60
atcttgctgg ccctagaatc ttaccaatcc cccttacaca tttccctaaa taactgtttt       120
tagcctttga aatacttgtt tcaaactttt aatagctaag gtcagtaatc tataggcttt       180
tatagtcttt gggtcaagat atacagtgtg tccacagctc atgttttcca gcctgtgtgg       240
gagcttggtc tgaagagaga ttaagtgata gcctacttat ggatcctgga gaattcttca       300
gaaatccatg tgtaactcag gtgcctattt ggttatgaca aaagatatgg ctaattttta       360
ttttgaaaag tttgaataaa cttagttttc tctttttcca cttgcaaaga gttttgatga       420
tggagactat tttcctgtgt ggggcacatg ccttggattt gaagagcttt cactgctgat       480
tagtggagag tgcttattaa ctgccacaga tactgttgac gtggcaatgc cgctgaactt       540
cactggaggt aagagtactc ttttcaaccc tttgaaaaat atttgctggg caggattagt       600
aagtagcaaa tatgaaagta aaagtgctta tttttatttt ctttttagcc ttttccctga       660
aggaaaaaat gcttatgaca ttagcattta aattatttac acaggttgaa gatgtgaatg       720
accatccttg tactcattta tgaggatgac ttaaggaatt gtgaatttta ataactcatt       780
gcttctagtg atatcaggac tgtgtgtgat gaagaactct gaaatgggat ctggttttga       840
ctcctagctt tgtcactttt accttgagga accattttac ctctctgagg ttcaatgtct       900
gcatttgcaa aaaggtctaa taacgcttag gggttttttt tgtgaatatt aaataagatt       960
tttttttttc tttttaagac agagtctctc tctgtcactc a                          1001
```

<210> 5
<211> 1001
<212> DNA
<213> Homo sapiens

<400> 5

```
cccttataga tggaaacttt cactgagtac aagctatata tccatgtatt taggaacaat        60
```

```
caacagtttt aacgaatagg aaccagtcat cttttctgcc ccggtggata ggcttagtgg      120

tggtcggtgg atggcgctga ccgcggttga tcagaggaag ctgacgccgt ttgcaggcgt      180

ggcctgggcg tgcccgcttc aaagtagacg cccggagttg gcccagttct gaccctagtg      240

atgcgcctgt gcgtgtgttc ctatcgccgt cccgtcttca cttcctacag atgccgcgac      300

cccccaaata gcaagtgtgg acattcacat tcaggggcac aggctgtccg tacagcgaac      360

gttgtcacca ttcccggaga tagggccccg accttcaagg gggcggtgcg gttggcgcgc      420

agttctcagg cgccttctag aatcccctgc cagcctcccc gctgcctggt taccaaactc      480

ggccggggac acccaacctc ccctctcgag gacctgggtt ggccagccca gctttgtacg      540

ggtgactgaa gtccacatcg acgtaacagt tgaaagggaa ggacgcggag accacccggt      600

acccgcccca ggcgtttcca gagtctccgc ttaggcggcc cgtgggggtc ggggtcgcgg      660

tcgcggtcgc ggaccgggga cagagcaatg gggcgggcgc gcagcgccgg ctgcactccc      720

agagcctccc accgccgccc cacggccgcc tagcgcgcgc cgggactgca tttcccatag      780

gcccacgctg cggagcaccg cccacccgag tctcacgcga gtgcgctgcc gcgaggtgtc      840

cctgccgcag cccccgcccg cccgcagagc ttttgaaagg cggcgggagg cggcgagcgc      900

catggccagt ccgggctgcc tgctgtgcgt gctgggcctg ctactctgcg gggcggcgag      960

cctcgagctg tctagacccc acggcgacac cgccaagaag c                          1001
```

<210> 6
<211> 1001
<212> DNA
<213> Homo sapiens

<400> 6

```
gcgcagttct caggcgcctt ctagaatccc ctgccagcct ccccgctgcc tggttaccaa        60
actcggccgg ggacacccaa cctcccctct cgaggacctg ggttggccag cccagctttg       120
tacgggtgac tgaagtccac atcgacgtaa cagttgaaag ggaaggacgc ggagaccacc       180
cggtacccgc cccaggcgtt tccagagtct ccgcttaggc ggcccgtggg ggtcggggtc       240
gcggtcgcgg tcgcggaccg gggacagagc aatggggcgg gcgcgcagcg ccggctgcac       300
tcccagagcc tcccaccgcc gccccacggc cgcctagcgc gcgccgggac tgcatttccc       360
ataggcccac gctgcggagc accgcccacc cgagtctcac gcgagtgcgc tgccgcgagg       420
tgtccctgcc gcagcccccg cccgcccgca gagcttttga aaggcggcgg gaggcggcga       480
gcgccatggc cagtccgggc tgcctgctgt gcgtgctggg cctgctactc tgcggggcgg       540
cgagcctcga gctgtctaga ccccacggcg acaccgccaa gaagcccatc atcggtaagg       600
aggctgtgcg gggccgcagc agacggggag gagctgggtg ttggcgctgg gcgcctcccg       660
ccgccgcgct ccagctggcg cgtcccgccg ggaaaaaccc ccagatgtgc ttcgcttttg       720
gtgcaaggag gaacccgggc tgggcaccat ttaggtcgta ctgaaatgta cctggttgag       780
gtacctcgtt cattccttta gactcatatg actcttaaag gtgggaggga ggagggtgca       840
ccatgaagaa tcctgcggat tttctttttt tgtacacagc taggaatttc tactaaaata       900
aaacatttcc ttttcgcaca tagttgggaa gttttactca tttacatcta caaattctgt       960
acatgcattg cattcatcaa aatgtattga tggcgtattt a                          1001
```

<210> 7
<211> 1001
<212> DNA
<213> Homo sapiens

<400> 7

```
aacgagatca cgccggtgct gtcgtactcc tacctggccg tgctggtgcc cgtgttcctg        60
ctcaccgact acctgcgcta cacgccggtg ctgctgctgc aggggctcag cttcgtgtcg       120
gtgtggctgc tgctgctgct gggccactcg gtggcgcaca tgcagctcat ggagctcttc       180
tacagcgtca ccatggccgc gcgcatcgcc tattcctcct acatcttctc tctcgtgcgg       240
cccgcgcgct accagcgtgt ggccggctac tcgcgcgctg cggtgctgct gggcgtgttc       300
accagctccg tgctgggcca gctgctggtc actgtgggcc gagtctcctt ctccacgctc       360
aactacatct cgctggcctt cctcaccttc agcgtggtcc tcgccctctt cctgaagcgc       420
cccaagcgca gcctcttctt caaccgcgac gaccgggggc ggtgcgaaac ctcggcttcg       480
gagctggagc gcatgaatcc tggcccaggc gggaagctgg gacacgccct gcgggtggcc       540
tgtggggact cagtgctggc gcggatgctg cgggagctgg gggacagcct gcggcggccg       600
cagctgcgcc tgtggtccct ctggtgggtc ttcaactcgg ccggctacta cctggtggtc       660
tactacgtgc acatcctgtg gaacgaggtg gaccccacca ccaacagtgc gcgggtctac       720
aacggcgcgg cagatgctgc ctccacgctg ctgggtacgc atgccctggt ccccgaggct       780
tcttcccgca gaggcttccc gtccacctcc gcctgccccg tggatccttc ccccggaggc       840
tccctctccc gcccccctgcc ccatggatac cttcccccag aggctctccg ccacctcccc       900
ctgccccgtg gatccttccc ccggaggctc cccctccccg ccccctgccc cgtggatcct       960
tcccccggag gctccccctc cccgccccct gccccgtgga t                          1001
```

<210> 8
<211> 1001
<212> DNA
<213> Homo sapiens

<400> 8

```
gcccagcagt gccatgagtc tagtgtggcc ccaaacccta aattttgtta gtttagttaa        60
taaagtaagt caattagtta attaacttgg ttttgattgg cacttaaatc actccatgtg       120
gctggagact tccctcagcc tcggagaccc tggaggtggt gacagcctgg cctgggggac       180
```

tccggagcag ctcaggtgtg ggtggctccc agtttggtgc tacggggtga ggatgggtct 240

ggggtctgca ttcgtctcca gggtggcgtt tggtcctgag tggcgaggag gccggcactg 300

ggcctcgttt tgcggggtag ggaggcctgc agaccatctt ccaaggtgcc ctgactccac 360

ccctccttcc aggcacagcg tcaccttcgt cccctccgga gctgcacgtg gcctgagcag 420

gatggtgccc tccagcccag cggtggagaa gcaggtgccc gtggaacctg ggcctgaccc 480

cgagctccgg tcctggcggc acctcgtgtg ctacctttgc ttctacggct tcatggcgca 540

gatacggcca ggggagagct tcatcacccc ctacctcctg ggcccgaca agaacttcac 600

gcgggagcag gcatgtgggt gccggggtgc cgggcggccg cctgtgggtg ggtgggcggg 660

gagcagcgtc tggaataggc acgtgggcgc cgggatgcgg gggcggccgc cagtgggtgg 720

gcagggagca gcttctggaa taggcacgtg ggcgctgggt gccagggcgg ctgcctgtgg 780

gtgggcgggg agcagcttct ggaataggca cgtgggtgcc ggggtgccgg gcggccgcct 840

gtgggtgggc ggggagcagc ttctggaata ggcatgtggg caccggggtg ctgggcggcc 900

gcctgtgggt gggcggggag cagcttctgg aataggcacg tgggtgctgg gtgccagggc 960

ggccgcctat gggtgggcgg ggagcagctt caggggccag g 1001

<210> 9
<211> 1001
<212> DNA
<213> Homo sapiens

<400> 9

```
agactggagt gcagtgactc aatcaggggt cactgcattc tttacttccc aagctcaagc      60
aatcttccca cctcagtcac ccgagtagct gggaccacag gcatgcacaa ccatgcccag     120
ctaatttttg tatttttgt agatacaggg tttcactatg ctgctcaggc tggtctcaaa      180
ctcctgggct caatcaacct gcctaggcct cccaaagggc tgggattaca ggccccacct     240
ggtctggtac ctaaactttc ttatgtgctt tactcctata gagaagaggc aaaacaaatt     300
attaactcca gaaaggaaaa gctggcaatg cagttttatt gaaattagct tgacatagtt     360
gctctggagc tcacagactt ctctcttctt ccccctgaag gtatggagag gttcaaaggc     420
caatatttcc atagccgcca atacaagcat ccagatggat ttgagggaaa acgcatcctg     480
gtgattggaa tgggaaactc aggctcagat attgctgttg agctgagtaa gaatgctgct     540
caggtgtgat gctctctgct taccatgtac ctggaggggga ggaagtgggg atgccatact    600
ggagaacccc agccatataa tcgcggctcc aatcctcatt aactagttgg ttggtagcgc     660
attgtggcat catagaaaat ctggaagtca agaaaccact ttacctccta gctctgtcaa     720
taaccagcca tgaatcctag agtgattcat ttcacttctc tgggagatgg ctccctcatt     780
tttaaaatgg gaacttttga ccagatgatt ttccatataa gaggcctttc atcaacatgg     840
ctcactgcag ccttgacctc ctgggctcca atcttcctgt catctcagcc tcctgagtag     900
ctgggactac aggcacatgc cacaccacac tcagctaatt ttcatatatt tgtagagatg     960
agggtcttgc catgttgccc agggtagtct caaactcctg a                        1001
```

<210> 10
<211> 1001
<212> DNA
<213> Homo sapiens

<400> 10

```
cggggatggt gatgggggac agagcaccac caacttgctc aaactttgcc aaaccaagag      60
ggatgacaga gttccttggc tttttatttt ctaacaataa acacgtaata aacattggtt     120
aaaagctaac agaggatctc atagtgtgcc agaaacactc tcttcctgtg actatttcag     180
ctttggttag atatccatct cccctgctct gttcccagga gcatgttgtc tgcacctctc     240
taatccctca gggcttggca cacttattca atgatggtga tcactgaatg gtcatgatca     300
tggctgactc tagtctaaaa tgaaatgagt ttcccatttc tattttgtaa gacatacgcc     360
tcacacatgt gtttgtttgc cttcttagtt tacaaagctt aatctagcct caccatgcag     420
tttatttcat gtttttggtg tttacttctc cctgcctttt tgcttttatg ttgcaggtgt     480
ttggaaagga ggtatgacac ggtatgtggt ttctgtagga aacacaaaac aactcttcgg     540
cacatcatct ggggcatttt attagcaggt aaataacaaa gatggttagg gataccactg     600
tctttttttt tttttttttc agggattccc ccaaatatta actttatgtg gaaaaagatt     660
aaacctttat tttctctggg acctcactga atcatcgggg gtggtagaga agtaaatcaa     720
gaagcccttt ataggggaag cctatgcaaa ttgtttccct accccgttta tgaggccaga     780
agctaatttt gtttttcttt gagatggagt ctagctctgt cttccaggct ggagtgcaat     840
ggcgccatct cagctcactg caacctctgc ctcccgggtt caagtgattc tcctgcctca     900
gcctcctgag tagctgggat tacaggcaca cgtcaccaca cccggctaat tttgtattt      960
ttagtagaga cagggtttca ctgtgttggc cgggctggtc t                        1001
```

<210> 11
<211> 1001
<212> DNA
<213> Homo sapiens

<400> 11

```
ggcatgactg gcttgaacct gggaggtggg ggttgcagtg ggccgagatc acgccactgc      60
cctccagcct gggcaacaga gtgagaccct atctcaaaaa aaaaaaaaga aaaaagaaaa     120
aagaaatgcg aagtctacag tgaagaagga agaagtgaag aacacatccg ctttgggagt     180
agccagtgat gtgggaaact gccttgccac tcccagaggg cagtatcttg tggctgcagg     240
```

```
gtttgctgtg ttttctggaa acagactttg ctcttgtgct ctgtccttcc catccctctc      300
aacttggggt cctgaatttt gctccaggac ttacaccaga ggatcgataa gttccgtttc      360
accccacctt tggaggacag ctgcttccac tatggcttca actccaacta cctgaagaaa      420
gtcatctcct actggcggaa tgaatttgac tggaagaagc aggtggagat tctcaacaga      480
taccctcact tcaagactaa gattgaaggt atgtttgcaa aacgccagcc agagagggat      540
gtatgtcatg agaacagcct tcttccacaa tgtgacttac agtcagataa agttggagag      600
attcagaacc caattatagg tgactgagat gtacttatac gttgtaacct tactaaatgc      660
aaaaatattg cagtcacaac aaatctgtcc atctttagag ctaggcagga acacatacat      720
gcaatttaaa aaccaaagaa atgcaccatc cagtccaatg atgtggcaat ctttgagagg      780
aaggatggag atgagattta gacccaggcc aagtggggtg gctcagaact gtaatcccag      840
cactttggga ggctgaggtg ggtagatcac gaggtcaaga gttcaagcag cctggccaag      900
atggtgaaat cccatctcta ctagaaatac aaaaaatagc caggtgtgtt ggcaggcagc      960
tgtaatccca gctactctgg aggctgaagc agagaattgc t                         1001
```

<210> 12
<211> 1001
<212> DNA
<213> Homo sapiens

<400> 12

gggaggattg cttgagcctg ggagttgagg ctgcagtgag ctgtgatgat gccactgtat 60
tccagcctgg gccacagagt gagaccctgt ctctaaaaaa agataaaaaa gatttgacac 120
ctgccaaaaa gaatttaccg attataaact gaggagaaaa gattaatctt ttttcataca 180
gattcaaata cgcagatgaa atcacatgcc aggtgaatgg taagagtttg taggaagtaa 240
gatgagtgaa gattggaatc ccagagactg aatctgtgga tttgctgaag agggcctcag 300
ggaagtgtta gactggaata ggtagagagg aggagaaaag acattccaga gaagcaaaat 360
aacaggaaca caatgaaaga aaaaaaaaac tgagggagaa gagctacttt ctgttttctc 420
cttctgcaag gcagttttgc tgtgattatt cctgtcctcc tccttgggac cactctgtcc 480
agagatctct tttctgaact gagaggctaa ctatccgcat cattaatgga atgcttgcct 540
cccaagcaga tagattctaa gttccctgaa ggcgtgacaa tctattcatt cttggcacag 600
tgaatagatt cgttgattca atccaccaaa ttcacttgca ttggctactc atgaactcat 660
catactatgt ctttgaagaa tgccttacgg ggtgtctaag ggagagaata gagtcatggg 720
ttctccattt ctgtttctga ctgggccaat aaagcccctt cctcatccct cttttccact 780
taccactagg gacagaaatt aaaaaccatg gcttcaggct actaaaagcc taaaccaaac 840
caaatcaaac caaacaacaa caacaaaata aggcaggttg gacaagctta cgagtgtaac 900

caaactatct ggaggtgtct gttgtgcggg acgcttattt ccactgacct aaatttccct 960
gccacttctc ccttaattta tcttgcctta ggatatttac a 1001

<210> 13
<211> 17
<212> DNA
<213> Artificial Sequence

<220>
<223> primer

<400> 13
cgcgccgctc tattcct 17

<210> 14
<211> 15
<212> DNA
<213> Artificial Sequence

<220>
<223> primer

<400> 14
ggccacgcgc tcaag 15

<210> 15
<211> 13

<212> DNA
<213> Artificial Sequence

<220>
<223> probe

<400> 15
cgcggcataa cga 13

<210> 16
<211> 13
<212> DNA
<213> Artificial Sequence

<220>
<223> probe

<400> 16
cgcggcgtaa cga 13

<210> 17
<211> 16
<212> DNA
<213> Artificial Sequence

<220>
<223> primer

<400> 17
gcctgacccc gagctc 16

<210> 18
<211> 24
<212> DNA
<213> Artificial Sequence

<220>
<223> primer

<400> 18
catgaagccg tagaagcaaa ggta 24

<210> 19
<211> 15
<212> DNA
<213> Artificial Sequence

<220>
<223> probe

<400> 19
acacgaggtg ccgcc 15

<210> 20
<211> 15
<212> DNA
<213> Artificial Sequence

<220>
<223> probe

<400> 20
acacgaggcg ccgcc 15

<210> 21
<211> 24
<212> DNA
<213> Artificial Sequence

<220>
<223> primer

<400> 21
tccagaaagg aaaagctggc aatg 24

<210> 22
<211> 23
<212> DNA
<213> Artificial Sequence

<220>
<223> primer

<400> 22
gagccatctc ccagagaagt gaa 23

<210> 23
<211> 20
<212> DNA
<213> Artificial Sequence

<220>
<223> primer

<400> 23
tgacatagtt gctctggagc 20